# FASCICULE DE BREVET EUROPEEN

(11) **EP 1 616 846 B1**
(45) Date de publication et mention de la délivrance du brevet: **14.10.2015**
(21) Numéro de dépôt: 05291382.9
(22) Date de dépôt: 27.06.2005
(51) Int. Cl.: C07C 2/10, C10G 50/00, C07C 11/02

(54) **Procede d'oligomerisation des olefines utilisant un catalyseur a base de silice-alumine**
Verfahren zur Oligomerisierung von Olefinen unter Verwendung von auf Siliciumdioxid-Aluminiumdioxid basierter Katalysator
Oligomerization process of olefins using a silica-alumina catalyst

(30) Priorité: 15.07.2004 FR 0407899
(43) Date de publication de la demande: 18.01.2006
(73) Titulaire: Institut Français du Pétrole, 92852 Rueil Malmaison Cedex (FR)
(72) Inventeur: Lacombe, Sylvie, 92400 COURBEVOIE (FR); Revel, Renaud, 38200 Serpaize (FR); Briot, Patrick, 38260 Pommier de Beaurepaire (FR); Llido, Eric, 69360 Communay (FR); Euzen, Patrick, 75001Paris (FR); Bobin, Carole, 69290 GRESIEU LA VARENNE (FR)

(56) Documents cités:
- EP-A- 0 550 922
- US-A- 5 051 386
- SASOL: "PURAL, CATAPAL High purity aluminas" janvier 2003 (2003-01), , XP002326387 Extrait de l'Internet: URL:http://www.sasol.com/sasol_internet/do wnloads/PURAL_CATAPAL_1055438108967.pdf> voir tableau dernière page entrée "CATAPAL B" * le document en entier *

## Description

### Domaine de l'invention

L'invention concerne tout procédé d'oligomérisation des oléfines permettant la production de carburant, par exemple la production d'essence et/ou de kérosène à partir de charges oléfiniques légères contenant entre 2 et 8 atomes de carbone, et en particulier à partir de charges oléfiniques légères contenant une forte proportion de propylène et/ou butènes utilisant un catalyseur d'oligomérisation à base de silice-alumine à teneur réduite en macropores. Par rapport aux catalyseurs de type silice-alumine déjà cités dans la littérature, le catalyseur de la présente invention présente une plus grande activité dans les procédés cités ci-dessus.

### Art Antérieur

Les procédés d'oligomérisation des oléfines légères destinés à produire des oléfines de plus haut poids moléculaire sont largement utilisés en raffinage et prétrochimie, dans le but de valoriser les oléfines légères en bases pour carburant de type essence, kérosène ou gazole, ou bien en solvant. Ces réactions d'oligomérisation sont conduites en présence d'un catalyseur, le plus souvent un catalyseur solide. Les oléfines se combinent en dimères, trimères, tétramères, etc., le degré d'oligomérisation dépendant du type de catalyseur utilisé et des conditions opératoires de température et de pression. L'avantage du procédé d'oligomérisation, par rapport à d'autres procédés bien connus dans le domaine du raffinage et de la pétrochimie conduisant à la même gamme de produits, réside dans le fait que les composés obtenus ne contiennent pas de soufre et contiennent très peu de composés aromatiques. Les catalyseurs d'oligomérisation solides souvent cités dans la littérature sont des catalyseurs de type acide phosphorique solide (par exemple US 2 913 506 et US 3 661 801), des silice-alumines (par exemple les brevets US 4 197 185, US 4 544 791 et EP 0 463 673), des zéolithes (par exemple les brevets US 4 642 404 et US 5 284 989) et, dans une moindre mesure, des hétéropolyanions (par exemple le brevet IN 170 903).

Les catalyseurs de type acide phosphorique solide présentent une bonne activité en oligomérisation, mais ils sont difficiles à manipuler, en particulier au moment du déchargement, car ils ont tendance à prendre en masse en présence d'oléfines. De plus, ils ne sont pas régénérables. Les catalyseurs de type hétéropolyanions conduisent à un degré de polymérisation limité, car ils supportent mal des températures élevées. Les zéolithes conduisent à des oligomères à taux de branchement plus limité que les catalyseurs précédents de par leur sélectivité de forme dans les micropores. Ceci est favorable pour la production de gazole devant présenter un indice de cétane correct mais peu favorable par exemple à la production d'essence devant présenter un bon indice d'octane. Enfin, les catalyseurs de type silice-alumines cités dans la littérature présentent des caractéristiques de porosité assez variables qui engendrent des réactivités différentes. Par exemple le brevet EP 0 463 673 revendique pour l'oligomérisation du propylène l'utilisation d'une silice-alumine amorphe avec un rapport molaire silice/ alumine entre 30/1 et 500/1, une surface spécifique entre 500 et 1000 m²/g, un volume poreux total entre 0,3 et 0,6 ml/g, un diamètre de pore moyen au plus égal à environ 1 nm, et aucun pore présentant un diamètre supérieur à 3 nm. Le brevet US 4 544 791 revendique pour l'oligomérisation d'oléfines en C4 l'utilisation d'une silice-alumine amorphe avec une teneur massique en silice entre 60 et 95 %, une surface spécifique entre 50 et 500 m²/g, un volume poreux total entre 0,4 et 0,9 ml/g, mais ladite silice-alumine ne présente pas de phase d'alumine en diffraction des rayons X.

EP 0 550 922 divulgue pour l'oligomérisation du propylène l'utilisation d'une silice-alumine amorphe présentant une phase d'alumine du type boehmite.

### Description de l'invention

Dans la suite du texte on entend par oligomérisation toute réaction d'addition d'une oléfine sur une autre oléfine.

La présente invention concerne un procédé d'oligomérisation des oléfines mettant en oeuvre un catalyseur silico-aluminique particulier, ledit catalyseur comprenant un support non-zéolithique à base de silice-alumine contenant une quantité supérieure à 5 % poids et inférieure ou égale à 95 % poids de silice (SiO₂) et possède les caractéristiques suivantes :
- un diamètre moyen poreux, mesuré par porosimétrie au mercure, compris entre 20 et 140 Å,
- un volume poreux total, mesuré par porosimétrie au mercure, compris entre 0,1 ml/g et 0,6 ml/g,
- un volume poreux total, mesuré par porosimétrie azote, compris entre 0,1 ml/g et 0,6 ml/g,
- une surface spécifique BET comprise entre 100 et 550 m²/g,
- un volume poreux, mesuré par porosimétrie au mercure, compris dans les pores de diamètre supérieurs à 140 Å inférieur à 0,1 ml/g,
- un volume poreux, mesuré par porosimétrie au mercure, compris dans les pores de diamètre supérieurs à 160 Å inférieur à 0,1 ml/g,
- un volume poreux, mesuré par porosimétrie au mercure, compris dans les pores de diamètre supérieurs à 200 Å inférieur à 0,1 ml/g,
- un volume poreux, mesuré par porosimétrie au mercure, compris dans les pores de diamètre supérieurs à 500 Å inférieur à 0,1 ml/g, de préférence inférieur à 0.05 ml/g, de manière très préférée inférieur à 0.02 ml/g et de manière encore plus préférée inférieur à 0.01 ml/g.
- un diagramme de diffraction X qui contient au moins les raies principales caractéristiques d'au moins une des alumines de transition comprise dans le groupe composé par les alumines alpha, rhô, khi, êta, gamma, kappa, thêta et delta, et le catalyseur silico-aluminique est obtenu à partir d'un mélange à quelque étape que ce soit d'un composé d'alumine partiellement soluble en milieu acide avec un composé de silice totalement soluble ou avec une combinaison totalement soluble d'alumine et de silice hydratée, mise en forme suivie d'un traitement hydrothermal ou thermique.

### Description du catalyseur d'oligomérisation

### Techniques de caractérisation

Dans l'exposé qui suit de l'invention, on entend par surface spécifique, la surface spécifique B.E.T. déterminée par adsorption d'azote conformément à la norme ASTM D 3663-78 établie à partir de la méthode BRUNAUER-EMMETT-TELLER décrite dans le périodique « The Journal of American Society", 60, 309, (1938).

Dans l'exposé qui suit de l'invention, on entend par "volume mercure des catalyseurs", le volume mesuré par intrusion au porosimètre à mercure selon la norme ASTM D4284-83 à une pression maximale de 4000 bar, utilisant une tension de surface de 484 dyne/cm et un angle de contact pour les catalyseurs silice-alumine amorphe de 140°. On définit le diamètre moyen mercure comme étant un diamètre tel que tous les pores de taille inférieure à ce diamètre constituent 50 % du volume poreux (V_{Hg}), dans un intervalle compris entre 36 Ǻ et 1000 Ǻ. L'angle de mouillage a été pris égal à 140° en suivant les recommandations de l'ouvrage "Techniques de l'ingénieur, traité analyse et caractérisation", pages 1050-5, de Jean Charpin et Bernard Rasneur".

Afin d'obtenir une meilleure précision, la valeur du volume mercure en ml/g donnée dans le texte qui suit correspond à la valeur du volume mercure total en ml/g mesurée sur l'échantillon moins la valeur du volume mercure en ml/g mesurée sur le même échantillon pour une pression correspondant à 30 psi (environ 2 bars). On définit également le diamètre moyen mercure comme étant un diamètre tel que tous les pores de taille inférieure à ce diamètre constituent 50 % du volume poreux total mercure.

Afin de mieux caractériser la distribution poreuse, on définit enfin les critères de distribution poreuse suivants en mercure : le volume V1 correspond au volume contenu dans les pores dont le diamètre est inférieur au diamètre moyen moins 30 Å. Le volume V2 correspond au volume contenu dans les pores de diamètre supérieur ou égal au diamètre moyen moins 30 Å et inférieur au diamètre moyen plus 30 Å. Le volume V3 correspond au volume contenu dans les pores de diamètre supérieur ou égal au diamètre moyen plus 30 Å. Le volume V4 correspond au volume contenu dans les pores dont le diamètre est inférieur au diamètre moyen moins 15 Å. Le volume V5 correspond au volume contenu dans les pores de diamètre supérieur ou égal au diamètre moyen moins 15 Å et inférieur au diamètre moyen plus 15 Å. Le volume V6 correspond au volume contenu dans les pores de diamètre supérieur ou égal au diamètre moyen plus 15 Å.

La distribution poreuse mesurée par adsorption d'azote a été déterminée par le modèle Barrett-Joyner-Halenda (BJH). L'isotherme d'adsorption-désorption d'azote selon le modèle BJH est décrit dans le périodique "The Journal of American Society", 73, 373, (1951) de E.P. Barrett, L.G. Joyner et P.P. Halenda. Dans l'exposé qui suit de l'invention, on entend par "volume adsorption azote", le volume mesuré pour P/P₀ = 0,99, pression pour laquelle il est admis que l'azote a rempli tous les pores. On définit le diamètre moyen désorption azote comme étant un diamètre tel que tous les pores inférieurs à ce diamètre constituent 50 % du volume poreux (Vp) mesuré sur la branche de désorption de l'isotherme azote.

Par "surface adsorption", on entend la surface mesurée sur la branche de l'isotherme d'adsorption. On se reportera par exemple à l'article de A. Lecloux dans Mémoires de la Société Royale des Sciences de Liège, 6ème série, Tome I, fasc.4, pp.169-209 (1971).

La teneur en sodium a été mesurée par spectrométrie d'absorption atomique.

La diffraction X est une technique pouvant être utilisée pour caractériser les catalyseurs selon l'invention. Dans l'exposé qui suit, l'analyse des rayons X est réalisée sur poudre avec un diffractomètre Philips PW 1830 opérant en réflexion et équipé d'un monochromateur arrière en utilisant la radiation CoKalpha (λK_{α1} = 1.7890 Å, λIK_{α2} = 1.793 Å, rapport d'intensité K_{α1}/ K_{α2} = 0.5). Pour le diagramme de diffraction X de l'alumine gamma, on se reportera à la base de données ICDD, fiche 10-0425. En particulier, les 2 pics les plus intenses sont situés à une position correspondant à un d compris entre 1,39 et 1,40 Å et un d compris entre 1,97 Å à 2,00 Å. On appelle d la distance inter-réticulaire qui est déduite de la position angulaire en utilisant la relation de Bragg (2 d ₍ₕₖ₁₎ * sin (θ) = n * λ). Par alumine gamma, on entend dans la suite du texte entre autres par exemple une alumine comprise dans le groupe composé des alumines gamma cubique, gamma pseudo-cubique, gamma tétragonale, gamma mal ou peu cristallisée, gamma grande surface, gamma basse surface, gamma issue de grosse boehmite, gamma issue de boehmite cristallisée, gamma issue de boehmite peu ou mal cristallisée, gamma issue d'un mélange de boehmite cristallisée et d'un gel amorphe, gamma issue d'un gel amorphe, gamma en évolution vers delta. Pour les positions des pics de diffraction des alumines êta, delta et thêta, on peut se référer à l'article de B.C. Lippens, J.J. Steggerda, dans Physical and Chemical Aspects of Adsorbents and Catalysts, E.G. Linsen (Ed.), Academic Press, London. 1970, p.171-211.

Pour les catalyseurs selon l'invention, le diagramme de diffraction X met en évidence un pic large caractéristique de la présence de silice amorphe.

Par ailleurs, dans l'ensemble du texte qui suit, le composé d'alumine peut contenir une fraction amorphe difficilement détectable par les techniques de DRX. On sous-entendra donc par la suite que les composés d'alumine utilisés ou décrits dans le texte peuvent contenir une fraction amorphe ou mal cristallisée.

Les catalyseurs selon l'invention ont été analysés par RMN MAS du solide de ²⁷Al sur un spectromètre de la firme Brüker de type MSL 400, en sonde 4 mm. La vitesse de rotation des échantillons est de l'ordre de 11 kHz. Potentiellement, la RMN de l'aluminium permet de distinguer trois types d'aluminium dont les déplacements chimiques sont reportés ci-après :
- entre 100 et 40 ppm, aluminiums de type tétra-coordinés, notés Al_{IV},
- entre 40 et 20 ppm, aluminiums de type penta-coordinés, notés Al_{V},
- entre 20 et - 100 ppm, aluminiums de type hexa-coordinés, notés Al_{VI}.

L'atome d'aluminium est un noyau quadripolaire. Dans certaines conditions d'analyse (champs de radiofréquence faible : 30 kHz, angle d'impulsion faible : π/2 et échantillon saturé en eau), la technique de RMN de rotation à l'angle magique (MAS) est une technique quantitative. La décomposition des spectres RMN MAS permet d'accéder directement à la quantité des différentes espèces. Le spectre est calé en déplacement chimique par rapport à une solution 1 M de nitrate d'aluminium. Le signal d'aluminium est à zéro ppm. On a choisi d'intégrer les signaux entre 100 et 20 ppm pour les Al_{IV} et Al_{V}, ce qui correspond à l'aire 1, et entre 20 et -100 ppm pour Al_{VI}, ce qui correspond à l'aire 2. Dans l'exposé qui suit de l'invention, on entend par proportion des Al_{VI} octaédriques le rapport suivant : aire 2/ (aire 1 + aire 2).

Une méthode de caractérisation des catalyseurs selon l'invention pouvant être utilisée est la microscopie électronique par transmission (MET). Pour cela on utilise un microscope électronique (du type Jeol 2010 ou Philips Tecnai20F éventuellement avec balayage) équipé d'un spectromètre à dispersion d'énergie (EDS) pour l'analyse des rayons X (par exemple un Tracor ou un Edax). Le détecteur EDS doit permettre la détection des éléments légers. L'association de ces deux outils, MET et EDS, permet de combiner l'imagerie et l'analyse chimique locale avec une bonne résolution spatiale.

Pour ce type d'analyse, les échantillons sont finement broyés à sec dans un mortier ; la poudre est ensuite incluse dans de la résine pour réaliser des coupes ultrafines d'épaisseur 70 nm environ. Ces coupes sont recueillies sur des grilles de cuivre recouvertes d'un film de carbone amorphe à trous servant de support. Elles sont ensuite introduites dans le microscope pour observation et analyse sous vide secondaire. En imagerie, on distingue alors aisément les zones d'échantillon des zones de résine. On procède ensuite à un certain nombre d'analyses, 10 au minimum, de préférence compris entre 15 et 30, sur différentes zones de l'échantillon industriel. La taille du faisceau électronique pour l'analyse des zones (déterminant approximativement la taille des zones analysées) est de 50 nm de diamètre au maximum, de préférence de 20 nm, de manière encore plus préférée 10, 5, 2 ou 1 nm de diamètre. En mode balayé, la zone analysée sera fonction de la taille de la zone balayée et non plus de la taille du faisceau généralement réduit.

Le traitement semi-quantitatif des spectres X recueillis à l'aide du spectromètre EDS permet d'obtenir la concentration relative de Al et de Si (en % atomique) et le rapport Si/Al pour chacune des zones analysées. On peut alors calculer la moyenne Si/Alₘ et l'écart type σ de cet ensemble de mesures. Dans les exemples non limitatifs de l'exposé qui suit de l'invention, la sonde de 50 nm est la sonde utilisée pour caractériser les catalyseurs selon l'invention sauf mention contraire.

La densité de remplissage tassée (DRT) est mesurée, comme cela est décrit dans l'ouvrage Applied Heterogeneous Catalysis de J.F. Le Page, J. Cosyns, P. Courty, E. Freund, J-P. Franck, Y. Jacquin, B. Juguin, C. Marcilly, G. Martino, J. Miquel, R. Montarnal, A. Sugier, H. Van Landeghem, Technip, Paris, 1987. On remplit un cylindre gradué de dimensions acceptables par additions successives et, entre deux additions successives, on tasse le catalyseur en secouant le cylindre jusqu'à atteindre un volume constant. Cette mesure est généralement réalisé sur 1000 cm³ de catalyseur tassé dans un cylindre dont le ratio hauteur sur diamètre est proche de 5:1. Cette mesure peut être, de manière préférée, réalisée sur des appareils automatisés tel que Autotap® commercialisé par Quantachrome®.

L'acidité de la matrice par est mesurée par IR. Les spectres IR sont enregistrées sur un interféromètre Nicolet de type Nexus-670 sous une résolution de 4 cm⁻¹ avec une apodisation de type Happ-Gensel. L'échantillon (20 mg) est pressé sous la forme d'une pastille auto-supportée est placé dans une cellule d'analyse in situ (25°C à 550°C, four déporté du faisceau IR, vide secondaire de 10-6 mbar). Le diamètre de la pastille est de 16 mm.

L'échantillon est prétraité de la façon suivante afin d'éliminer l'eau physisorbée et de déshydroxyler partiellement la surface du catalyseur pour avoir une image représentative de l'acidité du catalyseur en fonctionnement :
- montée en température de 25°C à 300°C en 3 heures
- palier de 10 heures à 300 °C
- descente de température de 300°C à 25°C en 3 heures

La sonde basique (pyridine) est ensuite adsorbée à pression saturante à 25°C puis thermo-désorbée selon les paliers suivants :
- 25°C pendant 2 heures sous vide secondaire
- 100°C 1 heure sous vide secondaire
- 200°C 1 heure sous vide secondaire
- 300°C 1 heure sous vide secondaire.

Un spectre est enregistré à 25°C à la fin du prétraitement et à chaque palier de désorption en mode transmission avec un temps d'accumulation de 100 s. Les spectres sont ramenés à iso-masse (donc supposée à iso-épaisseur) (20 mg exactement). Le nombre de sites de Lewis est proportionnel à la surface du pic dont le maximum se situe vers 1450 cm⁻¹, tout épaulement étant inclus. Le nombre de sites de Bronsted est proportionnel à la surface du pic dont le maximum se situe vers 1545 cm⁻¹. Le rapport du nombre de sites de Bronsted /nombre de sites de Lewis est estimé égal au rapport des surfaces de deux pics décrits ci-dessus. On utilise généralement la surface des pics à 25°C. Ce rapport B/L est de manière générale calculé à partir du spectre enregistré à 25°C à la fin du prétraitement.

### Caractéristiques du catalyseur d'oligomérisation des oléfines

Le catalyseur utilisé dans le procédé selon la présente invention est un catalyseur non-zéolitique à base de silice-alumine (c'est-à-dire comprenant silice et alumine), dont les caractéristiques sont les suivantes :
- La teneur massique en silice (SiO₂) supérieure à 5 % poids et inférieure ou égale à 95 % poids, de préférence comprise entre 10 et 80 % poids, de manière préférée supérieure à 20 % poids et inférieure à 80 % poids et de manière encore plus préférée supérieure à 25 % poids et inférieure à 75 % poids. La teneur en silice est avantageusement comprise entre 10 et 50 % poids.
- La teneur en impuretés cationiques est généralement inférieure à 0,1 %poids, de manière préférée inférieure à 0,05 % poids et de manière encore plus préférée inférieure à 0,025 % poids. On entend par teneur en impuretés cationiques la teneur totale en alcalins.
- La teneur en impuretés anioniques est généralement inférieure à 1 % poids, de manière préférée inférieure à 0,5 % poids et de manière encore plus préférée inférieure à 0,1 % poids.

La silice-alumine utilisée dans le procédé selon l'invention est de préférence une silice-alumine homogène à l'échelle du micromètre et dans laquelle la teneur en impuretés cationiques (par exemple Na⁺) est inférieure à 0,1 %poids, de manière préférée inférieure à 0,05 % poids et de manière encore plus préférée inférieure à 0,025 % poids et la teneur en impuretés anioniques (par exemple SO₄²⁻ ou Cl⁻) est inférieure à 1 % poids, de manière préférée inférieure à 0,5 % poids et de manière encore plus préférée inférieure à 0,1 % poids.

Ainsi tout procédé de synthèse de silice-alumine connu de l'homme du métier conduisant à une silice-alumine homogène à l'échelle du micromètre et dans lequel les impuretés cationiques (par exemple Na⁺) peuvent être ramenées à moins de 0,1 %, de manière préférée à une teneur inférieure à 0,05 % poids et de manière encore plus préférée inférieure à 0,025 % poids et dans lequel les impuretés anioniques (par exemple SO₄²⁻ ou Cl⁻) peuvent être ramenées à moins de 1 % et de manière plus préférée à une teneur inférieure à 0,05 % poids convient pour préparer les catalyseurs objet de l'invention.
- Le diamètre moyen poreux du catalyseur mesuré par porosimétrie au mercure, compris entre 20 et 140 Å, de préférence entre 40 et 120 Å et de manière encore plus préférée entre 50 et 100 Å,
- de préférence le rapport entre le volume V2, mesuré par porosimétrie au mercure, compris entre le D_{moyen} - 30 Å et le D_{moyen} +30 Å, sur le volume poreux total également mesuré par porosimétrie au mercure est supérieur à 0,6, de préférence supérieur à 0,7 et de manière encore plus préférée supérieur à 0,8.
- de préférence le volume V3 compris dans les pores de diamètres supérieurs à D_{moyen} +30 Å, mesuré par porosimétrie au mercure, est inférieur à 0,1 ml/g, de manière préférée inférieur à 0,06 ml/g et de manière encore plus préférée inférieur à 0,04 ml/g.
- de préférence le rapport entre le volume V5 compris entre le D_{moyen} -15 Å et le D_{moyen} +15 Å mesuré par porosimétrie au mercure, et le volume V2 compris entre le D_{moyen} -30 Å et le D_{moyen} +30 Å, mesuré par porosimétrie au mercure, est supérieur à 0,6, de manière préférée supérieur à 0,7 et de manière encore plus préférée supérieur à 0,8.
- de préférence le volume V6 compris dans les pores de diamètres supérieurs à D_{moyen} +15 Å, mesuré par porosimétrie au mercure, est inférieur à 0,2 ml/g, de manière préférée inférieur à 0,1 ml/g et de manière encore plus préférée inférieur à 0,05 ml/g.
- le volume poreux total, mesuré par porosimétrie au mercure, est compris entre 0,1 ml/g et 0,6 ml/g, de manière préférée compris entre 0,20 et 0,50 ml/g,
- le volume poreux total, mesuré par porosimétrie azote, compris entre 0,1 ml/g et 0,6 ml/g, de préférence compris entre 0,20 et 0,50 ml/g,
- la surface spécifique BET est comprise entre 100 et 550 m²/g, de préférence comprise entre 150 et 500 m²/g,
- le volume poreux, mesuré par porosimétrie au mercure, compris dans les pores de diamètre supérieurs à 140 Å est inférieur à 0,1 ml/g, de préférence inférieur à 0,05 ml/g et de manière encore plus préférée inférieur à 0,03 ml/g.
- le volume poreux, mesuré par porosimétrie au mercure, compris dans les pores de diamètre supérieurs à 160 Å est inférieur à 0,1 ml/g de préférence inférieur à 0,05 ml/g et de manière encore plus préférée inférieur à 0,025 ml/g.
- le volume poreux, mesuré par porosimétrie au mercure, compris dans les pores de diamètre supérieurs à 200 Å est inférieur à 0,1 ml/g, de préférence inférieur à 0,05 ml/g et de manière encore plus préférée inférieur à 0,025 ml/g.
- le volume poreux, mesuré par porosimétrie au mercure, compris dans les pores de diamètre supérieurs à 500 Å est inférieur à 0,1 ml/g, de préférence inférieur à 0.05 ml/g, de manière plus préférée inférieur à 0.02 ml/g et de manière encore plus préférée inférieur à0.01 ml/g.
- le diagramme de diffraction X du catalyseur contient au moins les raies principales caractéristiques d'au moins une des alumines de transition comprises dans le groupe composé par les alumines rhô, khi, kappa, êta, gamma, thêta et delta et de manière préférée au moins les raies principales caractéristiques d'au moins une des alumines de transition compris dans le groupe composé par l'alumine gamma, êta, thêta et delta, et de manière plus préférée au moins les raies principales caractéristiques de l'alumine gamma et êta, et de manière encore plus préférée le diagramme contient les pics à un d compris entre 1,39 à 1,40 Å et à un d compris entre 1,97 Å à 2,00 Å.

Les spectres RMN MAS du solide de ²⁷Al des catalyseurs montrent deux massifs de pics distincts. Un premier type d'aluminium dont le maximum résonne vers 10 ppm s'étend entre -100 et 20 ppm. La position du maximum suggère que ces espèces sont essentiellement de type Al_{VI} (octaédrique). Un deuxième type d'aluminium minoritaire dont le maximum résonne vers 60 ppm s'étend entre 20 et 110 ppm. Ce massif peut être décomposé en au moins deux espèces. L'espèce prédominante de ce massif correspondrait aux atomes d'Al_{IV} (tétraédrique). Pour les catalyseurs utilisés dans le procédé de la présente invention, avantageusement, la proportion des Al_{VI} octaédriques est supérieure à 50 %, de manière préférée supérieure à 60 %, et de manière encore plus préférée supérieure à 70 %.

Dans un mode de réalisation de l'invention, le catalyseur contient au moins deux zones silico-aluminiques, les dites zones ayant des rapports Si/Al inférieurs ou supérieurs au rapport Si/Al global déterminé par fluorescence X. Ainsi un catalyseur ayant un rapport Si/Al égal à 0,5 comprend par exemple deux zones silico-aluminiques, l'une des zones a un rapport Si/Al déterminé par MET inférieur à 0,5 et l'autre zone a un rapport Si/Al déterminé par MET compris entre 0,5 et 2,5.

Dans un autre mode de réalisation de l'invention, le catalyseur contient une seule zone silico-aluminique, ladite zone ayant un rapport Si/AI égal au rapport Si/Al global déterminé par fluorescence X et inférieur à 6.

L'acidité du catalyseur utilisé dans le procédé selon l'invention peut être de manière avantageuse, sans que cela ne restreigne la portée de l'invention, mesurée par suivi IR de la thermo-désorption de la pyridine. Généralement, le rapport B/L, tel que décrit ci-dessus, du catalyseur utilisé dans le procédé selon l'invention est compris entre 0.05 et 6, de manière préférée entre à 0,5 et 2.

Le catalyseur peut éventuellement contenir au moins un élément métallique choisi dans le groupe IVB (par exemple titane, zirconium, hafnium), le groupe VB (par exemple vanadium, niobium, tantale), le groupe VIB (par exemple chrome, molybdène, tungstène) ainsi que la première série du groupe VIII (Fe, Co, Ni) de la Classification Périodique des Éléments.

La teneur de ces métaux peut aller jusqu'à 10 % en poids du catalyseur final.

Le catalyseur peut éventuellement contenir également du silicium comme élément dopant déposé sur le support.

### Procédés de préparation du catalyseur silico-aluminique utilisé dans le procédé selon l'invention

Le demandeur a découvert que les catalyseurs silico-aluminiques obtenus à partir d'un mélange à quelque étape que ce soit d'un composé d'alumine partiellement soluble en milieu acide avec un composé de silice totalement soluble ou avec une combinaison totalement soluble d'alumine et de silice hydratées, mise en forme suivie d'un traitement hydrothermal ou thermique afin de l'homogénéiser à l'échelle micrométrique, voire à l'échelle nanométrique permettait d'obtenir un catalyseur particulièrement actif dans les procédés d'oligomérisation selon l'invention. Par "partiellement soluble en milieu acide", le demandeur entend que la mise en contact du composé d'alumine avant toute addition du composé de silice totalement soluble ou de la combinaison avec une solution acide par exemple d'acide nitrique ou d'acide sulfurique provoque leur dissolution partielle.

### Sources de silice

Les composés de silice utilisés selon l'invention peuvent avoir été choisis dans le groupe formé par l'acide silicique, les sols d'acide silicique, les silicates alcalins hydrosolubles, les sels cationiques de silicium, par exemple le métasilicate de sodium hydraté, le Ludox® sous forme ammoniacale ou sous forme alcaline, les silicates d'ammonium quaternaire. Le sol de silice peut être préparé selon l'une des méthodes connues de l'homme du métier. De manière préférée, une solution d'acide orthosilicique décationisée est préparée à partir d'un silicate alcalin hydrosoluble par échange ionique sur une résine.

### Sources de silice-alumines totalement solubles

Les silice-alumines hydratées totalement solubles utilisées selon l'invention peuvent étre préparées par coprécipitation vraie en conditions opératoires stationnaires maîtrisées (pH, concentration, température, temps de séjour moyen) par réaction d'une solution basique contenant le silicium, par exemple sous forme de silicate de sodium, optionnellement de l'aluminium par exemple sous forme d'aluminate de sodium avec une solution acide contenant au moins un sel d'aluminium par exemple le sulfate d'aluminium. Au moins un carbonate ou encore du CO₂ peut éventuellement être rajouté au milieu réactionnel.

Par coprécipitation vraie, le demandeur entend un procédé par lequel au moins un composé d'aluminium totalement soluble en milieu basique ou acide comme décrit ci-après, au moins un composé de silicium comme décrit ci-après sont mis en contact, simultanément ou séquentiellement, en présence d'au moins un composé précipitant et/ou coprécipitant de façon à obtenir une phase mixte essentiellement constituée de silice-alumine hydratée laquelle est éventuellement homogénéisée par agitation intense, cisaillement, broyage colloïdal ou encore par combinaison de ces opérations unitaires. Par exemple, ces silices-alumines hydratées peuvent avoir été préparées selon les enseignements des brevets américains US 2 908 635 ; US 3 423 332, US 3 433 747, US 3 451 947, US 3 629 152 et US 3 650 988.

La dissolution totale du composé de silice ou de la combinaison a été évaluée de manière approchée selon la méthode suivante. Une quantité fixée (15 g) du composé de silice ou de la combinaison hydratée est introduite dans un milieu de pH préétabli. De manière préférée, la concentration de solide rapporté par litre de suspension est 0,2 mole par litre. Le pH de la solution de dispersion est au moins de 12 et il peut être obtenu par utilisation d'une source alcaline. De manière préférée, il est intéressant d'utiliser NaOH. Le mélange est ensuite agité mécaniquement par un agitateur à turbine de type défloculeuse pendant 30 minutes à 800 t/min. Une fois l'agitation terminée, le mélange est centrifugé 10 minutes à 3000 t/min. Le gâteau est séparé du liquide surnageant. La solution a été filtré sur un filtre de porosité 4 de diamètre 19 cm. On procède ensuite au séchage puis à la calcination à 1000°C des 2 fractions. Puis, on définit un rapport R égal en divisant la masse décantée par la masse de solide en suspension. Par totalement soluble, on entend un rapport R au moins supérieur à 0,9.

### Sources d'alumine

Les composés d'alumine utilisés selon l'invention sont partiellement solubles en milieu acide. Ils sont choisis tout ou en partie dans le groupe des composés d'alumine de formule générale Al₂O₃, nH₂O. On peut en particulier utiliser des composés hydratés d'alumine tels que : l'hydrargillite, la gibbsite, la bayerite, la boehmite, la pseudo-boehmite et les gels d'alumine amorphe ou essentiellement amorphe. On peut également mettre en oeuvre les formes déshydratées de ces composés qui sont constitués d'alumines de transition et qui comportent au moins une des phases prises dans le groupe : rhô, khi, êta, gamma, kappa, thêta, et delta, qui se différencient essentiellement par l'organisation de leur structure cristalline. L'alumine alpha appelée communément corindon peut être incorporée dans une faible proportion dans le catalyseur selon l'invention.

Cette propriété de dissolution partielle est une propriété recherchée de l'invention, elle s'applique aux poudres d'alumine hydratées, aux poudres atomisées d'alumine hydratées, aux dispersions ou suspensions d'alumine hydratées ou à l'une quelconque de leur combinaison, avant une quelconque addition d'un composé contenant tout ou en partie du silicium.

La dissolution partielle du composé d'alumine a été évaluée de manière approchée selon la méthode suivante. Une quantité précise du composé d'alumine en poudre ou en suspension est introduite dans un milieu de pH préétabli. Le mélange est ensuite agité mécaniquement. Une fois, l'agitation terminée, le mélange est laissé sans agitation durant 24 heures. De manière préférée, la concentration de solide en Al₂O₃ rapportée par litre de suspension est 0,5 mole par litre. Le pH de la solution de dispersion est de 2 et est obtenu soit par utilisation de HNO₃, soit de HCl, soit de HClO₄. De manière préférée, il est intéressant d'utiliser HNO₃. La répartition des fractions sédimentées et dissoutes a été suivie par dosage de l'aluminium par absorption UV. Les surnageants ont été ultrafiltrés (membrane de polyéther-sulfones, Millipore NMWL 30 000) et digérés dans de l'acide concentré. La quantité d'aluminium dans le surnageant correspond au composé d'alumine non-sédimentée et à l'aluminium dissous et la fraction ultrafiltrée à l'aluminium dissous uniquement. La quantité de particules sédimentées est déduite de la concentration théorique en aluminium dans la dispersion (en considérant que tout le solide introduit est dispersé) et des quantités de boehmite réellement dispersées et d'aluminium en solution.

Les précurseurs d'alumine utilisés selon la présente invention se distinguent donc de ceux utilisés dans le cas des co-précipitations vraies, qui sont entièrement solubles en milieu acide : sels cationiques d'alumine, par exemple le nitrate d'aluminium. Les méthodes faisant partie de l'invention se distinguent des co-précipitations vraies car l'un des éléments, en l'occurrence le composé d'aluminium, est partiellement soluble.

Pour mettre en oeuvre l'alumine, tout composé d'alumine de formule générale Al₂O₃, nH₂O peut être utilisé. Sa surface spécifique est comprise entre 150 et 600 m²/g. On peut en particulier utiliser des composés hydratés d'alumine tels que : l'hydrargillite, la gibbsite, la bayerite, la boehmite, la pseudo-boehmite et les gels d'alumine amorphe ou essentiellement amorphe. On peut également mettre en oeuvre les formes déshydratées de ces composés qui sont constitués d'alumines de transition et qui comportent au moins une des phases prises dans le groupe : rhô, khi, êta, gamma, kappa, thêta, delta et alpha, qui se différencient essentiellement par l'organisation de leur structure cristalline. Lors de traitements thermiques, ces différentes formes sont susceptibles d'évolution entre elles, selon une filiation complexe qui dépend des conditions opératoires du traitement. On peut également utiliser dans des proportions mesurées l'alumine alpha appelée communément corindon.

L'hydrate d'aluminium Al₂O₃, nH₂O utilisé de manière plus préférentielle est la boehmite, la pseudo-boehmite et les gels d'alumine amorphe ou essentiellement amorphe. Un mélange de ces produits sous quelque combinaison que ce soit peut être également utilisé.

La boehmite est généralement décrite comme un monohydrate d'aluminium de formule Al₂O₃, nH₂O qui englobe en réalité un large continuum de matériaux de degrés d'hydratation et d'organisation variables avec des frontières plus ou moins bien définies : la boehmite gélatineuse la plus hydratée, avec n pouvant être supérieur à 2, la pseudo-boehmite ou la boehmite micro-cristalline avec n compris entre 1 et 2, puis la boehmite cristalline et enfin la boehmite bien cristallisée en gros cristaux avec n voisin de 1. La morphologie du monohydrate d'aluminium peut varier dans de larges limites entre ces deux formes extrêmes, aciculaire et prismatique. Tout un ensemble de formes variables peut être utilisé entre ces deux formes : chaîne, bateaux, plaquettes entrelacées.

La préparation et/ou la mise en forme de l'hydrate d'aluminium peuvent ainsi constituer la première étape de la préparation de ces catalyseurs. De nombreux brevets relatent la préparation et/ou la mise en forme de supports à base d'alumine de transition issues de monohydrate d'aluminium : US 3 520 654, US 3 630 670, US 3 864 461, US 4 154 812, US 4 313 923, DE 3 243 193 et US 4 371 513.

Des hydrates d'aluminium relativement purs peuvent être utilisés sous forme de poudre, amorphes ou cristallisés ou cristallisés contenant une partie amorphe. L'hydrate d'aluminium peut également être introduit sous forme de suspensions ou dispersions aqueuses. Les suspensions ou dispersions aqueuses d'hydrate d'aluminium mise en oeuvre selon l'invention peuvent être gélifiables ou coagulables. Les dispersions ou suspensions aqueuses peuvent également être obtenues ainsi qu'il est bien connu de l'homme du métier par peptisation dans l'eau ou l'eau acidulée d'hydrates d'aluminium.

La dispersion d'hydrate d'aluminium peut être réalisée par tout procédé connu de l'homme du métier : dans un réacteur en "batch", un mélangeur en continu, un malaxeur, un broyeur colloïdal. Un tel mélange peut être également réalisé dans un réacteur à écoulement piston et, notamment dans un mélangeur statique. On peut citer les réacteurs Lightnin.

En outre, on peut également mettre en oeuvre comme source d'alumine une alumine ayant été soumise au préalable à un traitement susceptible d'améliorer son degré de dispersion. A titre d'exemple, on pourra améliorer la dispersion de la source d'alumine par un traitement d'homogénéisation préliminaire. Par homogénéisation, on peut utiliser au moins un des traitements d'homogénéisation décrit dans le texte qui suit.

Les dispersions ou suspensions aqueuses d'alumine que l'on peut mettre en oeuvre sont notamment les suspensions ou dispersions aqueuses de boehmites fines ou ultra-fines qui sont composés de particules ayant des dimensions dans le domaine colloïdal.

Les boehmites fines ou ultra-fines mises en oeuvre selon la présente invention peuvent notamment avoir été obtenues selon les brevets FR 1 261 182 et FR 1 381 282 ou selon la demande de brevet européen EP 15 196.

On peut également mettre en oeuvre les suspensions ou dispersions aqueuses obtenues à partir de pseudo-boehmite, de gels d'alumine amorphe, de gels d'hydroxyde d'aluminium ou d'hydrargillite ultra-fine.

Le monohydrate d'aluminium peut être acheté parmi une variété de sources commerciales d'alumine telle que notamment les PURAL®, CATAPAL®, DISPERAL®, DISPAL® commercialisée par la société SASOL ou encore HIQ® commercialisée par ALCOA, ou selon les méthodes connues de l'homme du métier : elle peut être préparée par déshydratation partielle de trihydrate d'aluminium par des méthodes conventionnelles ou elle peut être préparée par précipitation. Lorsque ces alumines se présentent sous forme d'un gel, elles sont peptisées par l'eau ou une solution acidulée. Dans la précipitation, la source acide peut être par exemple au moins un des composés suivants : le chlorure d'aluminium, le sulfate d'aluminium et le nitrate d'aluminium. La source basique d'aluminium peut être choisie parmi les sels basiques d'aluminium tels que l'aluminate de sodium et l'aluminate de potassium.

Comme agents précipitants, l'hydroxyde de sodium, le carbonate de sodium, la potasse et l'ammoniaque peuvent être utilisés. Les agents précipitants sont choisis de telle manière que la source d'alumine selon la présente invention et ces agents soient précipités ensemble.

Selon la nature acide ou basique du composé de départ à base d'aluminium, on précipite l'hydrate d'aluminium à l'aide d'une base ou d'un acide choisi par exemple parmi l'acide chlorhydrique, l'acide sulfurique, la soude ou un composé basique ou acide de l'aluminium tel que cités ci-dessus. Les deux réactifs peuvent être le sulfate d'aluminium et l'aluminate de soude. Pour un exemple de préparation de alpha-monohydrate d'aluminium utilisant le sulfate d'aluminium et l'aluminate de soude, on peut se référer notamment au brevet US 4 154 812.

La pseudo-boehmite peut notamment avoir été préparée selon le procédé décrit dans le brevet US 3 630 670 par réaction d'une solution d'aluminate alcalin avec une solution d'un acide minéral. La pseudo-boehmite peut notamment avoir été préparée selon le procédé décrit dans le brevet US 3 630 670 par réaction d'une solution d'aluminate alcalin avec une solution d'un acide minéral. Elle peut également avoir été préparée comme décrit dans le brevet FR 1 357 830.

Les gels d'alumine amorphe peuvent notamment avoir été préparés selon les procédés décrits dans l'article "Alcoa Paper No.19 (1972)" pages 9 à 12 et notamment par réaction d'aluminate d'acide ou d'un sel d'aluminium ou par hydrolyse d'alcoolates d'aluminium ou par hydrolyse de sels basiques d'aluminium.

Les gels d'hydroxyde d'aluminium peuvent notamment être ceux qui ont été préparés selon les procédés décrits dans les brevets américains US 3 268 295 et US 3 245 919.

Les gels d'hydroxyde d'aluminium peuvent notamment être ceux préparés selon les procédés décrits dans le brevet WO 00/01 617, par mélange d'une source acide d'aluminium et d'une base ou d'une source basique d'aluminium et d'un acide de manière à précipiter un monohydrate d'alumine, les étapes suivantes étant :
2. mûrissement,
3. filtration,
4. lavage et
5. séchage,
procédés caractérisés en ce que le mélange de l'étape une est réalisé sans rétro-mélange.

L'hydrargilite ultra-fine peut notamment avoir été préparée selon le procédé décrit dans le brevet US 1 371 808, par évolution à une température comprise entre la température ambiante et 60°C de gels d'alumine sous forme de gâteau et contenant 0,1 ions acides monovalents par rapport à l'alumine compté en molécules d'Al₂O₃.

On peut également mettre en oeuvre les suspensions ou dispersions aqueuses de boehmite ou de pseudo-boehmite ultra-pures préparées selon un procédé dans lequel on effectue la réaction d'un aluminate alcalin avec de l'anhydride carbonique pour former un précipité d'hydroxycarbonate d'aluminium amorphe, on sépare le précipité obtenu par filtration puis on lave celui-ci (le procédé est notamment décrit dans le brevet US 3 268 295).

Ensuite,
a) dans une première étape, on mélange le précipité lavé d'hydroxycarbonate d'aluminium amorphe avec une solution acide, d'une base ou d'un sel ou de leurs mélanges ; ce mélange est effectué en versant la solution sur l'hydroxycarbonate, le pH du milieu ainsi constitué étant inférieur à 11,
b) dans une deuxième étape, on chauffe le milieu réactionnel ainsi constitué à une température inférieure à 90°C pendant un temps d'au moins 5 minutes et
c) dans une troisième étape, on chauffe le milieu résultant de la deuxième étape à une température comprise entre 90°C et 250°C.

Les dispersions ou suspensions de boehmite et pseudo-boehmite obtenus selon ce procédé présentent une teneur en alcalins inférieure à 0,005 % exprimée sous forme de rapport pondéral oxyde du métal alcalin /Al₂O₃.

Lorsqu'on désire fabriquer des supports de catalyseurs très purs, on utilise de préférence des suspensions ou dispersions de boehmites ou de pseudo-boehmites ultra-pures qui ont été obtenues selon le procédé décrit ci-dessus, ou les gels d'hydroxyde d'aluminium qui ont été préparés à partir de l'hydrolyse des alcoolates d'aluminium selon un procédé du type décrit dans le brevet US 2 892 858.

On décrit sommairement le procédé de fabrication qui conduit à de tels gels d'hydroxyde d'aluminium de type boehmite obtenue comme sous-produit dans la fabrication de l'alcool par hydrolyse d'un alcoolate ou alcoxyde d'aluminium (synthèse de Ziegler). Les réactions de synthèse d'alcools Ziegler sont décrites notamment dans le brevet US 2 892 858. Selon ce procédé, on prépare tout d'abord le triéthylaluminium à partir d'aluminium, d'hydrogène et d'éthylène, la réaction étant réalisée en deux étapes avec recyclage partiel du triéthylaluminium.

On ajoute de l'éthylène dans l'étape de polymérisation et on oxyde ensuite le produit obtenu en alcoolate d'aluminium, les alcools étant obtenus par hydrolyse.

Les gels d'hydroxyde d'aluminium peuvent également être ceux qui ont été préparés selon les procédés décrits dans les brevets américains US 4 676 928 et US 6 030 599.

L'alumine hydratée obtenue comme sous-produit de la réaction de Ziegler est notamment décrite dans un bulletin de la société CONOCO du 19 janvier 1971.

La dimension des particules d'alumine constituant la source d'alumine peut varier dans de larges limites. Elle est généralement comprise entre 1 et 100 microns.

### Méthodes de préparation du catalyseur

Le catalyseur utilisé dans le procédé selon l'invention peut être avantageusement préparé par l'une des méthodes décrites ci-après.

A titre d'exemple, une méthode de préparation d'une silice-alumine faisant partie de l'invention consiste à préparer à partir d'un silicate alcalin hydrosoluble une solution d'acide orthosilicique (H₂SiO₄, H₂O) décationisée par échange ionique puis à l'ajouter simultanément à un sel cationique d'aluminium en solution, par exemple le nitrate, et à de l'ammoniaque dans des conditions opératoires contrôlées ; ou encore ajouter la solution d'acide orthosilicique au sel cationique d'aluminium en solution et à coprécipiter la solution obtenue obtenue par de l'ammoniaque dans des conditions opératoires contrôlées conduisant à un produit homogène. Cet hydrogel de silice-alumine est mélangé avec de la poudre ou une suspension d'hydrate d'aluminium. Après filtration et lavage, séchage avec mise en forme puis calcination préférentiellement sous air, en four rotatif, à température élevée et pendant un temps suffisant pour favoriser les interactions entre l'alumine et la silice, généralement au moins 2 heures, un catalyseur répondant aux caractéristiques de l'invention est obtenu.

Une autre méthode de préparation de silice-alumine selon l'invention consiste à précipiter l'hydrate d'alumine comme ci-avant, à le filtrer et le laver, puis à le mélanger avec l'acide orthosilicique aqueux de façon à obtenir une suspension, laquelle est intimement homogénéisée par forte agitation et cisaillement. Une turbine Ultraturrax ou encore une turbine Staro peut être utilisée, ou encore un broyeur colloïdal par exemple, un broyeur colloïdal Staro. La suspension homogène est alors séchée par atomisation comme ci-avant, puis calcinée entre 500 et 1200°C pendant au moins 3 heures : un catalyseur silice-alumine utilisable dans le procédé selon l'invention est obtenu.

Une autre méthode faisant partie de l'invention consiste à préparer comme ci-avant une solution décationisée d'acide orthosilicique puis à l'ajouter simultanément ou consécutivement à un composé d'alumine, par exemple un hydrate d'aluminium en poudre ou en suspension acidulée. Afin d'augmenter le diamètre des pores du catalyseur silice-alumine final, au moins un composé basique peut éventuellement être rajouté au milieu réactionnel. Après une homogénéisation poussée de la suspension par agitation, ajustement éventuel par filtration de la teneur en matière sèche puis éventuellement ré-homogénéisation, le produit est séché avec mise en forme simultanément ou consécutivement, puis calciné comme ci-avant.

Une autre méthode faisant également partie de l'invention consiste à préparer une suspension ou une dispersion aqueuse d'alumine, par exemple un monohydrate d'aluminium puis à l'ajouter simultanément ou consécutivement à un composé de silice, par exemple un silicate de sodium. Afin d'augmenter le diamètre des pores du catalyseur silice-alumine final, au moins un composé basique peut éventuellement être rajouté au milieu réactionnel. Le catalyseur est obtenu par filtration et lavage, éventuellement lavage par une solution ammoniacale pour extraire par échange ionique le sodium résiduel, séchage avec mise en forme simultanément ou consécutivement. Après séchage avec mise en forme puis calcination comme ci-avant, un catalyseur répondant aux caractéristiques de l'invention est obtenu. La taille des particules d'alumine utilisée est de préférence comprise entre 1 et 100 microns pour obtenir une bonne homogénéisation du catalyseur silice-alumine selon l'invention.

Pour augmenter le diamètre des mésopores du catalyseur silice-alumine, il peut être particulièrement avantageux comme l'enseigne le brevet US 4 066 574 de préparer une suspension ou une dispersion aqueuse d'alumine, par exemple un monohydrate d'aluminium puis de neutraliser par une solution basique, par exemple de l'ammoniaque, puis à l'ajouter simultanément ou consécutivement à un composé de silice par exemple une solution décationisée d'acide orthosilicique. Après une homogénéisation poussée de la suspension par agitation intense, ajustement éventuel par filtration de la teneur en matière sèche puis ré-homogénéisation, le produit est séché avec mise en forme simultanément ou consécutivement, puis calciné comme ci-avant. Cette méthode fait également partie des méthodes utilisées selon l'invention.

Dans l'exposé des méthodes précitées, on emploie le terme "homogénéisation" pour décrire la remise en solution d'un produit contenant une fraction solide par exemple une suspension, une poudre, un précipité filtré, puis sa dispersion sous agitation intense. L'homogénéisation d'une dispersion est un procédé bien connu de l'homme du métier. Ladite homogénéisation peut être réalisée par tout procédé connu de l'homme du métier, par exemple dans un réacteur en batch, un mélangeur en continu ou un malaxeur. Un tel mélange peut être réalisé dans un réacteur à écoulement piston et notamment dans un réacteur statique. On peut citer les réacteurs Lightnin. Une turbine Ultraturrax® ou encore une turbine Staro® peut être utilisée, ou encore un broyeur colloïdal par exemple, un broyeur colloïdal Staro. Les broyeurs colloïdaux commerciaux IKA® peuvent être aussi utilisés.

Dans l'ensemble des méthodes précitées, il peut être éventuellement souhaitable d'ajouter, lors d'une étape quelconque de la préparation, une proportion mineure d'au moins un élément stabilisant choisi dans le groupe formé par la zircone et le titane. L'élément stabilisant est de préférence ajouté sous forme d'un sel soluble.

### Mise en forme des catalyseurs

Le catalyseur peut être obtenu par mise en forme de la silice-alumine par toute technique connue de l'homme du métier. La mise en forme peut être réalisée par exemple par extrusion, par pastillage, par la méthode de la coagulation en goutte (oil-drop), par granulation au plateau tournant ou par toute autre méthode bien connue de l'homme du métier.

La mise en forme peut également être réalisée en présence des différents constituants du catalyseur et extrusion de la pâte minérale obtenue, par pastillage, mise en forme sous forme de billes au drageoir tournant ou au tambour, coagulation en goutte, oil-drop, oil-up, ou tout autre procédé connu d'agglomération d'une poudre contenant de l'alumine et éventuellement d'autres ingrédients choisis parmi ceux mentionnés ci-dessus.

Les catalyseurs utilisés selon l'invention ont la forme de sphères ou d'extrudés. Il est toutefois avantageux que le catalyseur se présente sous forme d'extrudés d'un diamètre compris entre 0,5 et 5 mm et plus particulièrement entre 0,7 et 2,5 mm. Les formes sont cylindriques (qui peuvent être creuses ou non), cylindriques torsadés, multilobées (2, 3, 4 ou 5 lobes par exemple), ou anneaux. La forme cylindrique est utilisée de manière préférée, mais toute autre forme peut être utilisée.

Par ailleurs, ces catalyseurs mis en oeuvre selon la présente invention peuvent avoir été traités ainsi qu'il est bien connu de l'homme du métier par des additifs pour faciliter la mise en forme et/ou améliorer les propriétés mécaniques finales des catalyseurs silico-aluminiques. A titre d'exemple d'additifs, on peut citer notamment la cellulose, la carboxyméthyl-cellulose, la carboxyéthyl-cellulose, du tall-oil, les gommes xanthaniques, des agents tensio-actifs, des agents floculants comme les polyacrylamides, le noir de carbone, les amidons, l'acide stéarique, l'alcool polyacrylique, l'alcool polyvinylique, des biopolymères, le glucose, les polyéthylènes glycols, etc.

Le réglage de la porosité caractéristique des catalyseurs de l'invention est opéré partiellement lors de cette étape de mise en forme des particules de catalyseurs.

La mise en forme peut être réalisée en utilisant les techniques de mise en forme des catalyseurs, connues de l'homme du métier, telles que par exemple : extrusion, dragéification, séchage par atomisation ou encore pastillage.

On peut ajouter ou retirer de l'eau pour ajuster la viscosité de la pâte à extruder. Cette étape peut être réalisée à tout stade de l'étape de malaxage.

La teneur en acide ajouté au malaxage avant la mise en forme est inférieure à 30 %, de préférence comprise entre 0,5 et 20 % poids de la masse anhydre en silice et alumine engagée dans la synthèse.

L'extrusion peut être réalisée par n'importe quel outil conventionnel, disponible commercialement. La pâte issue du malaxage est extrudée à travers une filière, par exemple à l'aide d'un piston ou d'une mono-vis ou double vis d'extrusion. Cette étape d'extrusion peut être réalisée par toute méthode connue de l'homme de métier.

Les extrudés de catalyseur selon l'invention ont généralement une résistance à l'écrasement d'au moins 70 N/cm et de manière préférée supérieure ou égale à 100 N/cm.

### Séchage et calcination du catalyseur

Le séchage est effectué par toute technique connue de l'homme du métier.

Pour obtenir le catalyseur utilisé dans le procédé de la présente invention, il est préférable de calciner de préférence en présence d'oxygène moléculaire, par exemple en effectuant un balayage d'air, à une température inférieure ou égale à 1100°C. Au moins une calcination peut être effectuée après l'une quelconque des étapes de la préparation. Ce traitement par exemple peut être effectué en lit traversé, en lit léché ou en atmosphère statique. Par exemple, le four utilisé peut être un four rotatif tournant ou un four vertical à couches traversées radiales. Les conditions de calcination : température et durée dépendent principalement de la température maximale d'utilisation du catalyseur, les conditions préférées de calcination se situant entre plus d'une heure à 200°C et moins d'une heure à 1100°C. La calcination peut être opérée en présence de vapeur d'eau. La calcination finale peut être éventuellement effectuée en présence d'une vapeur acide ou basique. Par exemple, la calcination peut être réalisée sous pression partielle d'ammoniaque.

### Traitements post-synthèse

Des traitements post-synthèse peuvent être effectués, de manière à améliorer les propriétés du support, notamment son homogénéité telle que définie précédemment.

Selon un mode de réalisation préféré, le traitement post-synthèse est un traitement hydrothermal. Le traitement hydrothermal est effectué par toute technique connue de l'homme du métier. Par traitement hydrothermal, on entend mise en contact à n'importe quelle étape de l'élaboration du support mixte avec de l'eau en phase vapeur ou en phase liquide. Par traitement hydrothermal, on peut entendre notamment mûrissement, vapotraitement, autoclavage, calcination sous air humide, réhydratation. Sans que cela réduise la portée de l'invention, un tel traitement a pour effet de rendre mobile le composant silice.

Selon l'invention, le mûrissement peut avoir lieu avant ou après la mise en forme. Selon l'invention, le traitement de vapotraitement se fait dans un four en présence de vapeur d'eau. La température pendant le vapotraitement peut être comprise entre 600 et 1100 °C et de préférence supérieure à 700°C pendant une période de temps comprise entre 30 minutes et 3 heures. La teneur en vapeur d'eau est supérieure à 20 g d'eau par kg d'air sec et de préférence supérieure à 40 g d'eau par kg d'air sec et de manière préférée supérieure à 100 g d'eau par kg d'air sec. Un tel traitement peut, le cas échéant, remplacer totalement ou en partie le traitement de calcination.

Selon l'invention, le support peut ainsi être éventuellement soumis à un traitement hydrothermal en atmosphère confinée. On entend par traitement hydrothermal en atmosphère confinée un traitement par passage à l'autoclave en présence d'eau sous une température supérieure à la température ambiante.

Au cours de ce traitement hydrothermal, on peut traiter de différentes manières la silice-alumine mise en forme. Ainsi, on peut imprégner d'acide la silice-alumine, préalablement à son passage à l'autoclave, l'autoclavage de la silice-alumine étant fait soit en phase vapeur, soit en phase liquide, cette phase vapeur ou liquide de l'autoclave pouvant être acide ou non. Cette imprégnation, préalable à l'autoclavage, peut être acide ou non. Cette imprégnation, préalable à l'autoclavage peut être effectuée à sec ou par immersion de la silice-alumine dans une solution aqueuse acide. Par imprégnation à sec, on entend mise en contact de l'alumine avec un volume de solution inférieur ou égal au volume poreux total de l'alumine traitée. De préférence, l'imprégnation est réalisée à sec.

L'autoclave est de préférence un autoclave à panier rotatif tel que celui défini dans la demande de brevet EP 0 387 109.

La température pendant l'autoclavage peut être comprise entre 100 et 250°C pendant une période de temps comprise entre 30 minutes et 3 heures.

Une méthode préférée selon l'invention consiste à déposer du silicium sur le catalyseur mis en forme sur le précurseur calciné ou non, de préférence calciné. Pour cela, on utilisera par exemple une solution d'un composé du silicium de type silicone ou émulsion d'huile silicone qui sera imprégnée sur la précurseur préformé. Par la suite, il sera possible de procéder à un séchage par exemple à 120°C, et de procéder à une calcination par exemple et de façon préférée sous air en lit traversé, par exemple à 500°C pendant 4 heures.

De nombreuses sources de silicium peuvent être employées. Ainsi, on peut utiliser l'orthosilicate d'éthyle Si(OEt)₄, les siloxanes, les polysiloxanes, les silicones, les émulsions de silicones, les silicates d'halogénures comme le fluorosilicate d'ammonium (NH₄)₂SiF₆ ou le fluorosilicate de sodium Na₂SiF₆. Le silicium peut être ajouté par exemple par imprégnation de silicate d'éthyle en solution dans un mélange eau/alcool. Le silicium peut être ajouté par exemple par imprégnation d'un composé du silicium de type silicone ou l'acide silicique mis en suspension dans l'eau.

On peut également éventuellement ajouter à la silice-alumine, à quelque étape que ce soit de la préparation des précurseurs de métaux de transition choisi dans le groupe IVB (titane, zirconium, hafmium), le groupe VI (Vanadium, Niobium, Tantale), le groupe VIB (Chrome, Molybdène, Tungstène) ainsi que la première série du groupe VIII (Fe, Co, Ni). La teneur de ces métaux peut aller jusqu'à 10 % en poids du catalyseur final.

### Description du procédé d'oligomérisation

Le procédé selon l'invention est un procédé d'oligomérisation des oléfines permettant la production de carburant, par exemple la production d'essence et/ou de kérosène à partir de charges oléfiniques légères contenant entre 2 et 8 atomes de carbone, et en particulier à partir de charges oléfiniques légères contenant une forte proportion de propylène et/ou butènes utilisant un catalyseur d'oligomérisation à base de silice-alumine à teneur réduite en macropores.

L'invention concerne donc un procédé d'oligomérisation des oléfines dans lequel le catalyseur d'oligomérisation comprend un support non-zéolithique à base de silice-alumine contenant une quantité supérieure à 5 % poids et inférieure ou égale à 95 % poids de silice (SiO₂) et possède les caractéristiques suivantes :
- un diamètre moyen poreux, mesuré par porosimétrie au mercure, compris entre 20 et 140 Å,
- un volume poreux total, mesuré par porosimétrie au mercure, compris entre 0,1 ml/g et 0,6 ml/g,
- un volume poreux total, mesuré par porosimétrie azote, compris entre 0,1 ml/g et 0,6 ml/g,
- une surface spécifique BET comprise entre 100 et 550 m²/g,
- un volume poreux, mesuré par porosimétrie au mercure, compris dans les pores de diamètre supérieurs à 140 Å inférieur à 0,1 ml/g,
- un volume poreux, mesuré par porosimétrie au mercure, compris dans les pores de diamètre supérieurs à 160 Å inférieur à 0,1 ml/g,
- un volume poreux, mesuré par porosimétrie au mercure, compris dans les pores de diamètre supérieurs à 200 Å inférieur à 0,1 ml/g,
- un volume poreux, mesuré par porosimétrie au mercure, compris dans les pores de diamètre supérieurs à 500 Å inférieur à 0, 1 ml/g, de préférence inférieur à 0.05 ml/g, de manière très préférée inférieur à 0.02 ml/g et de manière encore plus préférée inférieur à 0.01 ml/g et
- un diagramme de diffraction X qui contient au moins les raies principales caractéristiques d'au moins une des alumines de transition comprise dans le groupe composé par les alumines alpha, rhô, khi, êta, gamma, kappa, thêta et delta.

Par rapport aux catalyseurs de l'art antérieur, le catalyseur selon la présente invention présente une activité accrue dans les procédés d'oligomérisation des oléfines cités ci-dessous, ainsi qu'une grande facilité de mise en oeuvre.

### Charges

Les oléfines peuvent provenir par exemple d'une unité de craquage catalytique et/ou d'une unité de vapocraquage, et/ou d'une unité de déshydrogénation de paraffines et/ou d'une unité de déshydratation polymérisante de méthanol en eau et oléfines légères et/ou de toute autre source d'oléfines légères.

La coupe oléfinique envoyée dans le réacteur d'oligomérisation contenant le catalyseur selon l'invention est de préférence débarrassée d'impuretés, telles que par exemple l'eau, les dérivés soufrés, les dérivés azotés basiques, avant d'être introduite dans le réacteur d'oligomérisation.

La coupe oléfinique peut être une coupe C4 oléfinique, qui comprend habituellement à plus de 90 % poids de l'isobutane, du n-butane, du 1-butène, des 2-butènes, de l'isobutène et éventuellement une petite quantité de butadiène. Le butadiène est généralement éliminé en amont de l'oligomérisation par un procédé d'hydrogénation sélective.

La coupe oléfinique peut être une coupe C3-C4 oléfinique. La composition de la coupe C3-C4 oléfinique est très variable selon sa provenance. Elle peut comprendre entre environ 20 et 50% poids de propylène et propane, entre environ 50 et 80% poids de l'isobutane, du n-butane, du 1-butène, des 2-butènes, de l'isobutène, et éventuellement une petite quantité de butadiène. Le butadiène est généralement éliminé en amont de l'oligomérisation par un procédé d'hydrogénation sélective.

La coupe oléfinique peut être une coupe C3 oléfinique. Elle comprend habituellement au moins 90% poids de propylène , propane.

Dans tous les cas de procédés d'oligomérisation selon l'invention, l'exothermicité de la réaction peut être gérée par un recyclage d'au moins une partie de l'effluent non-converti, qui contient en particulier les paraffines qui n'ont pas été transformées lors de la réaction, vers le réacteur d'oligomérisation, et/ou la charge peut être diluée par un ajout de paraffines provenant d'une autre source, lesdites paraffines étant de même poids moléculaire et/ou plus lourdes que la charge oléfinique, lesdites paraffines étant aliphatiques ou cycliques.

Dans tous les cas de procédés conduisant à la formation d'essence et/ou de kérosène, et/ou plus généralement d'une coupe oléfinique avec un point d'ébullition commençant à une température supérieure à 150°C, les coupes oléfiniques précitées obtenues en sortie du procédé peuvent éventuellement être hydrogénées, partiellement ou totalement.

### Modes de réalisation

### Premier mode de réalisation : oligomérisation sélective

Le catalyseur de ladite invention est particulièrement performant dans le procédé où on soumet une coupe C4 oléfinique à une oligomérisation en limitant la conversion globale des n-butènes à moins de 10 %, de manière préférée à moins de 5 %, alors que plus de 90 % de la quantité d'isobutène est convertie, de préférence plus de 95 %. L'isobutène est converti à plus de 90 % en dimères et trimères. Ensuite l'effluent d'oligomérisation est soumis à une distillation de telle sorte qu'une des fractions récupérées (effluent léger) contient à plus de 90 % poids du butane, de l'isobutane et les butènes qui n'ont pas réagi lors de l'oligomérisation, une partie au moins de cette fraction alimentant ensuite par exemple une unité d'alkylation ou une unité d'hydratation, alors que l'autre fraction constituée des oligomères obtenus est utilisée comme base essence, éventuellement après hydrogénation partielle ou totale.

Le procédé cité ci-dessus sera appelé dans la suite du texte "oligomérisation sélective".

La réaction d'oligomérisation est effectuée à une température entre 30 et 300°C, sous une pression entre environ 0,1 et 20 MPa et le volume de charge hydrocarbonée envoyé par volume de catalyseur et par heure se situe entre 0,05 et 5 h⁻¹, De préférence la température se situe entre 40 et 160°C, et la pression entre 2 et 7 MPa, de façon à s'assurer que la réaction s'effectue en phase liquide, ou au moins en phase homogène, et le volume de charge hydrocarbonée envoyé par volume de catalyseur et par heure se situe de préférence entre 0,8 et 2,5 h⁻¹.

La technologie du réacteur peut être un lit fixe, un lit fluidisé ou un lit circulant. La technologie préférée est une mise en oeuvre en lit fixe.

De manière optionnelle, les oligomères ainsi obtenus peuvent être réinjectés dans un réacteur d'oligomérisation supplémentaire contenant par exemple le catalyseur d'oligomérisation précédemment décrit, de façon à augmenter la longueur de chaîne des oligomères et atteindre ainsi la coupe kérosène, ou plus généralement une coupe oléfinique avec un point d'ébullition commençant à une température supérieure à 150°C.

De manière optionnelle, l'effluent léger d'oligomérisation, c'est-à-dire la coupe C4, peut être introduit dans un réacteur d'hydroisomérisation visant à hydroisomériser une partie du 1-butène non-converti en 2-butène, de façon à se rapprocher de l'équilibre thermodynamique. Les autres constituants de l'effluent ne sont alors pas convertis de façon significative durant l'étape d'hydroisomérisation. La conversion du 1-butène en 2-butène est très utile si la fraction C4 ainsi obtenue peut ensuite être introduite dans un réacteur d'alkylation aliphatique sur acide fluorhydrique, les produits obtenus par alkylation du 2-butène avec l'isobutane ayant un meilleur indice d'octane que l'alkylat obtenu à partir du 1-butène.

Étant donné la forte exothermicité de la réaction d'oligomérisation, la quantité d'isobutène dans la charge hydrocarbonée alimentant le réacteur d'oligomérisation est de préférence inférieure à 35 % en poids, de manière encore plus préférée inférieure à 15 % en poids, ceci étant éventuellement obtenu en diluant la charge, par exemple avec du butane ou de l'isobutane ou du raffinat de l'unité d'oligomérisation.

### Deuxième mode de réalisation

Le catalyseur décrit dans ladite invention est également particulièrement performant dans le procédé consistant à soumettre une coupe C4 oléfinique ou C3-C4 oléfinique à une oligomérisation de telle sorte qu'une partie des butènes contenus dans la charge soit convertis en dimères ou trimères, utilisés ensuite comme base essence. Dans le cas de ce procédé, moins de 80 % des butènes est converti et au moins 80 %, de préférence au moins 90 %, de l'isobutène est converti. Ce procédé permet de maximiser la quantité d'essence tout en minimisant la quantité de kérosène formé.

Dans le réacteur d'oligomérisation, la température se situe entre 40 et 250°C, de préférence entre 50 et 200°C, et la pression se situe entre 0,1 et 10 MPa, de préférence entre 0,1 et 5 MPa, et la quantité de charge hydrocarbonée envoyée par volume de catalyseur et par heure se situe entre 0,05 et 5 h⁻¹, de préférence entre 0,8 et 2,5 h⁻¹. La technologie du réacteur peut être un lit fixe, un lit fluidisé ou un lit circulant. La technologie préférée met en oeuvre un lit fixe.

Une variante de ce mode de réalisation du procédé consiste à utiliser comme charge une charge oléfinique dans laquelle l'isobutène a été au préalable éliminé en partie ou totalement, par exemple en utilisant en amont de l'unité d'oligomérisation une unité d'éthérification en faisant réagir sélectivement l'isobutène avec un alcool, par exemple le méthanol ou l'éthanol, sans convertir le n-butène, ou bien en utilisant en amont de l'unité d'oligomérisation une unité d'oligomérisation sélective telle que celle décrite précédemment. Les oligomères produits présentent alors moins de branchements que ceux obtenus par traitement de la coupe complète incluant l'isobutène.

### Troisième mode de réalisation

Un troisième mode de réalisation du procédé selon l'invention consiste à soumettre une coupe C4 oléfinique, contenant éventuellement des traces de propylène, à une oligomérisation de telle sorte que la majeure partie des butènes contenus dans la charge soit convertie en dimères ou trimères, utilisés ensuite comme base essence. Dans le cas de ce procédé, au moins 90 % des 1-butène, au moins 80 % des 2-butènes, au moins 97 % de l'isobutène et au moins 80 % du propylène sont convertis. Ce procédé permet de maximiser la quantité d'essence sans fabriquer de kérosène.

La coupe C4 oléfinique comprend habituellement de l'isobutane, du n-butane, du 1-butène, du 2-butène, de l'isobutène et éventuellement un petite quantité de butadiène. Le butadiène est généralement éliminé en amont de l'oligomérisation par un procédé d'hydrogénation sélective, pour éviter des réactions de polymérisation qui rendraient le catalyseur inactif.

Ledit procédé comprend les étapes suivantes :
- première étape d'oligomérisation : on traite une coupe C4 ou C3-C4 oléfinique, dans un premier réacteur d'oligomérisation dans lequel la conversion globale des n-butènes contenus dans la charge est inférieure à 45 % et la conversion de l'isobutène est supérieure à 80 %, de préférence supérieure à 85 %, les oligomères obtenus étant des dimères et trimères à plus de 80 % ;
- on envoie l'effluent de la première étape d'oligomérisation dans une colonne de fractionnement de façon à récupérer une première fraction contenant l'isobutène et les n-butènes non-convertis et une deuxième fraction consistant à 90 % en dimères et trimères de la réaction d'oligomérisation ;
- deuxième étape d'oligomérisation : la première fraction récupérée est introduite dans un deuxième réacteur d'oligomérisation dans lequel les oléfines sont converties en grande partie en dimères et trimères, c'est-à-dire qu'au moins 50 % des n-butènes sont convertis, de préférence au moins 75 % du 1-butène et au moins 55 % du 2-butène sont convertis ; et
- on envoie l'effluent de la deuxième étape d'oligomérisation dans la colonne de fractionnement associée au premier réacteur d'oligomérisation ou dans une deuxième colonne pour séparer l'essence ou le kérosène des composés en C4 non-convertis.

Dans les réacteurs d'oligomérisation, la température se situe entre 40 et 250°C, de préférence entre 45 et 200°C, et la pression se situe entre 0,1 et 10 MPa, de préférence entre 0,1 et 5 MPa, et la quantité de charge hydrocarbonée envoyée par volume de catalyseur et par heure se situe entre 0,05 et 5 h⁻¹, de préférence entre 0,8 et 2,5 h⁻¹. La technologie du réacteur peut être un lit fixe, un lit fluidisé ou un lit circulant. De préférence la technologie est un lit fixe.

De préférence, dans le deuxième réacteur d'oligomérisation, les conditions opératoires sont plus sévères que dans le premier réacteur.

Le même procédé peut être appliqué à une charge C3C4 oléfinique.

### Quatrième mode de réalisation

Le catalyseur de ladite invention est également particulièrement performant dans le procédé consistant à soumettre une coupe C4 oléfinique ou une coupe C3C4 oléfinique, à une oligomérisation de telle sorte que la majeure partie des butènes contenus dans la charge soit convertis, de façon à former une base essence et une base kérosène. Dans le cas de ce procédé, au moins 90 % des 1-butène, au moins 80 % des 2-butènes et au moins 97 % de l'isobutène sont convertis. La coupe C4 oléfinique comprend habituellement essentiellement de l'isobutane, du n-butane, du 1-butène, du 2-butène, de l'isobutène et éventuellement une petite quantité de butadiène. La coupe C3C4 oléfinique comprend en plus du propane et du propylène.

Dans le réacteur d'oligomérisation, la température se situe entre 60 et 250°C, de préférence entre 100 et 200°C, et la pression se situe entre 0,1 et 10 MPa, de préférence entre 0,1 et 5 MPa, et la quantité de charge hydrocarbonée envoyée par volume de catalyseur et par heure se situe entre 0,05 et 5 h⁻¹, de préférence entre 0,8 et 2,5 h⁻¹.

La technologie du réacteur peut être un lit fixe, un lit fluidisé ou un lit circulant. De préférence la technologie est un lit fixe.

### Cinquième mode de réalisation

Le catalyseur de ladite invention est également particulièrement performant dans le procédé dans lequel on soumet une coupe C3 oléfinique à une oligomérisation de telle sorte que la majeure partie du propylène contenu dans la charge soit convertie, c'est à dire qu'au moins 80 % du propylène contenu dans la charge est converti, de façon à former une base essence et une base kérosène. La coupe C3 oléfinique comprend habituellement au moins 90% poids de propylène , propane.

La réaction d'oligomérisation est effectuée à une température entre 30 et 300°C, sous une pression entre environ 0,1 et 20 MPa et le volume de charge hydrocarbonée envoyé par volume de catalyseur et par heure se situe entre 0,05 et 5 h-1, De préférence la température se situe entre 40 et 160°C, et la pression entre 2 et 7 MPa, le volume de charge hydrocarbonée envoyé par volume de catalyseur et par heure se situe de préférence entre 0,8 et 2,5 h-1.

La technologie du réacteur peut être un lit fixe, un lit fluidisé ou un lit circulant. La technologie préférée est une mise en oeuvre en lit fixe.

Les exemples suivants illustrent la présente invention sans toutefois en limiter la portée.

### Exemple 1 : Silice-alumine non-conforme à l'invention (SA1)

Les caractéristiques du catalyseur SA1 sont les suivantes :
- La composition du catalyseur en silice-alumine est de 89,3 % Al₂O₃ et de 10,7 % SiO₂.
- La surface BET est de 370 m²/g.
- Le volume poreux total, mesuré par adsorption d'azote, est de 0,457 ml/g.
- Le diamètre poreux moyen, mesuré par porosimétrie au mercure, est de 48 Å.
- Le rapport entre le volume V2, mesuré par porosimétrie au mercure, compris entre le D_{moyen} -30 Å et le D_{moyen} +30 Å sur le volume mercure total est de 0,92.
- Le volume V3, mesuré par porosimétrie au mercure, compris dans les pores de diamètres supérieurs à D_{moyen} +30 Å est de 0,042ml/g.
- Le volume V6, mesuré par porosimétrie au mercure, compris dans les pores de diamètres supérieurs à D_{moyen} +15 Å est de 0,013 ml/g.
- Le volume poreux, mesuré par porosimétrie au mercure, compris dans les pores de diamètre supérieurs à 140 Å est de 0,02 ml/.
- Le volume poreux, mesuré par porosimétrie au mercure, compris dans les pores de diamètre supérieurs à 160 Å est de 0,02 ml/g.
- Le volume poreux, mesuré par porosimétrie au mercure, compris dans les pores de diamètre supérieurs à 200 Å est 0,01 ml/g.
- Le volume poreux, mesuré par porosimétrie au mercure, est compris dans les pores de diamètre supérieurs à 500 Å est de 0,004 ml/g.
- Le diagramme de diffraction X ne contient pas les raies principales caractéristiques de l'alumine gamma.
- Le rapport B/L du catalyseur est de 1.
- La densité de remplissage tassée du catalyseur est de 0,72 g/cm³.
- La teneur en sodium atomique est de 300 +/- 20 ppm. La teneur en soufre atomique est de 2500 ppm.

Les spectres RMN MAS du solide de ²⁷Al des catalyseurs montrent deux massifs de pics distincts. Un premier type d'aluminium dont le maximum résonne vers 10 ppm s'étend entre -100 et 20 ppm. La position du maximum suggère que ces espèces sont essentiellement de type Al_{VI} (octaédrique). Un deuxième type d'aluminium majoritaire dont le maximum résonne vers 60 ppm s'étend entre 20 et 100 ppm. L'espèce prédominante de ce massif correspondrait aux atomes d'Al_{IV} (tétraédrique). La proportion des Al_{VI} octaédriques est de 35 %.

Le catalyseur contient une seule zone silico-aluminique avec un rapport Si/Al déterminé par microsonde en MET de 4,3.

### Exemple 2 : Préparation et mise en forme d'une silice-alumine conforme à l'invention (SA2)

La poudre d'hydroxyde d'aluminium a été préparée selon le procédé décrit dans le brevet WO 00/01 617. La taille des particules moyenne des particules d'hydroxyde d'aluminium mesurée par granulométrie laser est de 40 microns. Cette poudre est mélangée à un sol de silice préparé par échange sur résine décationisante, puis filtré sur résine de porosité 2. Les concentrations en sol de silice et en poudre d'hydroxyde d'aluminium sont ajustées de manière à obtenir une composition finale de 70 % Al₂O₃ et de 30 % SiO₂. La mise en forme est réalisée en présence de 15 % d'acide nitrique par rapport au produit anhydre. Le malaxage se fait sur un malaxeur bras en Z. L'extrusion est réalisée par passage de la pâte au travers d'une filière munie d'orifices de diamètre 1,4 mm. Les extrudés ainsi obtenus sont séchés à 150°C, puis calcinés à 550°C.

Les caractéristiques du catalyseur sont les suivantes :
- La composition du catalyseur en silice-alumine est de 85,3 % Al₂O₃ et de 14,7 % SiO₂.
- La surface BET est de 430 m²/g.
- Le volume poreux total, mesuré par adsorption d'azote, est de 0,24 ml/g.
- Le diamètre poreux moyen, mesuré par porosimétrie au mercure, est de 46 Å.
- Le rapport entre le volume V2, mesuré par porosimétrie au mercure, compris entre le D_{moyen} -30 Å et le D_{moyen} +30 Å sur le volume mercure total est de 0,7.
- Le volume V3, mesuré par porosimétrie au mercure, compris dans les pores de diamètre supérieur à D_{moyen} +30 Å est de 0,07 ml/g.
- Le volume V6, mesuré par porosimétrie au mercure, compris dans les pores de diamètre supérieur à D_{moyen} +15 Å est de 0,08 ml/g,
- Le volume poreux, mesuré par porosimétrie au mercure, compris dans les pores de diamètre supérieur à 140 Å est de 0,06 ml/g,
- Le volume poreux, mesuré par porosimétrie au mercure, compris dans les pores de diamètre supérieur à 160 Å est de 0,051 ml/g.
- Le volume poreux, mesuré par porosimétrie au mercure, compris dans les pores de diamètre supérieur à 200 Å est de 0,047 ml/g.
- Le volume poreux, mesuré par porosimétrie au mercure, est compris dans les pores de diamètre supérieur à 500 Å est de 0,03 ml/g.
- Le rapport B/L du catalyseur est de 1,1.
- La densité de remplissage tassée du catalyseur est de 0,80 g/cm³.
- Le diagramme de diffraction X contient les raies principales caractéristiques de l'alumine gamma et notamment il contient les pics à un d compris entre 1,39 à 1,40 Å et à un d compris entre 1,97 Å à 2,00 Å.
- La teneur en sodium atomique est de 40 +/- 20 ppm. La teneur en soufre atomique est de 200 ppm.

Les spectres RMN MAS du solide de ²⁷Al des catalyseurs montrent deux massifs de pics distincts. Un premier type d'aluminium dont le maximum résonne vers 10 ppm s'étend entre -100 et 20 ppm. La position du maximum suggère que ces espèces sont essentiellement de type Al_{VI} (octaédrique). Un deuxième type d'aluminium minoritaire dont le maximum résonne vers 60 ppm et qui s'étend entre 20 et 100 ppm. Ce massif peut être décomposé en au moins deux espèces. L'espèce prédominante de ce massif correspondrait aux atomes d'Al_{IV} (tétraédrique). La proportion des Al_{VI} octaédriques est de 65 %.

Le catalyseur contient deux zones silico-aluminiques, les dites zones ayant des rapports Si/Al inférieurs ou supérieurs au rapport Si/Al global déterminé par fluorescence X. L'une des zones a un rapport Si/Al déterminé par MET de 4.35 et l'autre zone a un rapport Si/AI déterminé par MET de 1,75.

### Exemple 3: Evaluation catalytique des silice-alumine SA1 et SA2 dans un procédé d'oligomérisation avec conversion poussée

Une coupe C4 oléfinique issue d'une unité de vapocraquage est soumise à un traitement d'hydrogénation sélective dans le but d'éliminer le butadiène, puis séchée sur un tamis moléculaire de type 13X pour éliminer les traces de soufre et d'eau.

La composition de la charge à l'issue de ces traitements est la suivante :

| Composition de la charge (% poids) | |
|---|---|
| Isobutane | 1,54 |
| n-butane | 7,74 |
| isobutène | 39,89 |
| 1-butène | 28,64 |
| Σ 2-butènes | 22,18 |

Cette charge est envoyée dans un réacteur isotherme d'oligomérisation contenant le catalyseur à base de silice-alumine SA1 ou SA2. Les conditions opératoires sont les suivantes :

| Catalyseur | SA1 | SA2 |
|---|---|---|
| Pression | 6,0 MPa | 6,0 MPa |
| Température | 130°C | 130°C |
| VVH | 2 h⁻¹ | 2 h⁻¹ |

En sortie de réacteur d'oligomérisation la composition massique de l'effluent est la suivante :

| Catalyseur | SA1 | SA2 |
|---|---|---|
| Composition de l'effluent (% poids) | | |
| Isobutane | 2,56 | 3,64 |
| n-butane | 7,89 | 7,89 |
| isobutène | - | - |
| 1-butène | 0,72 | 0,26 |
| Σ 2-butènes | 12,35 | 6,81 |
| polymère C5+ | 76,48 | 81,40 |

L'utilisation du catalyseur SA2 conduit à un rendement en polymère C5+ plus important qu'avec le catalyseur SA1.

### Exemple 4: Évaluation catalytique des silice-alumine SA1 et SA2 dans un procédé d'oligomérisation avec conversion modérée

Une coupe C4 oléfinique issue d'une unité de vapocraquage est soumise à un traitement d'hydrogénation sélective destiné à en éliminer le butadiène, puis séchée sur un tamis moléculaire de type 13X pour éliminer les traces de soufre et d'eau. La composition de la charge à l'issue de ces traitements est la suivante :

| Composition de la charge (% poids) | |
|---|---|
| Isobutane | 1,50 |
| n-butane | 6,63 |
| isobutène | 49,48 |
| 1-butène | 27,86 |
| Σ 2-butènes | 14,53 |

En sortie de réacteur d'oligomérisation la composition massique de l'effluent est la suivante :

| Catalyseur | SA1 | SA2 |
|---|---|---|
| Pression | 2,5 MPa | 2,0 MPa |
| Température | 85°C | 80°C |
| WH | 0,5 h⁻¹ | 0,5 h⁻¹ |

L'utilisation du catalyseur SA2 conduit à un rendement en polymère C5+ plus important qu'avec le catalyseur SA1.

| Catalyseur | SA1 | SA2 |
|---|---|---|
| Composition de l'effluent (% poids) | | |
| Isobutane | 1,61 | 1,57 |
| n-butane | 6,60 | 6,64 |
| isobutène | 0,48 | 0,41 |
| 1-butène | 23,41 | 22,90 |
| Σ 2-butènes | 18,92 | 19,01 |
| polymère C5+ | 48,97 | 49,47 |

### Exemple 5: Evaluation catalytique des silice-alumine SA1 et SA2 dans un procédé d'oligomérisation avec conversion poussée

Une coupe C4 oléfinique issue d'une unité de craquage catalytique est soumise à un traitement d'hydrogénation sélective dans le but d'éliminer le butadiène, puis séchée sur un tamis moléculaire de type 13X pour éliminer les traces de soufre et d'eau.

La composition de la charge à l'issue de ces traitements est la suivante :

| Composition de la charge (% poids) | |
|---|---|
| Isobutane | 29,10 |
| n-butane | 11,45 |
| isobutène | 14,22 |
| 1-butène | 14,20 |
| Σ 2-butènes | 31,03 |

Cette charge est envoyée dans un réacteur isotherme d'oligomérisation contenant le catalyseur à base de silice-alumine SA1 ou SA2. Les conditions opératoires sont les suivantes :

| Catalyseur | SA1 | SA2 |
|---|---|---|
| Pression | 6,0 MPa | 6,0 MPa |
| Température | 125°C | 125°C |
| VVH | 2 h⁻¹ | 2 h⁻¹ |

En sortie de réacteur d'oligomérisation la composition massique de l'effluent est la suivante :

| Catalyseur | SA1 | SA2 |
|---|---|---|
| Composition de l'effluent (% poids) | | |
| Isobutane | 29,30 | 29,42 |
| n-butane | 11,45 | 11,45 |
| isobutène | 0,31 | 0,08 |
| 1-butène | 4,03 | 2,41 |
| Σ 2-butènes | 36,25 | 23,15 |
| polymère C5+ | 18,66 | 33,49 |

L'utilisation du catalyseur SA2 conduit à un rendement en polymère C5+ plus important qu'avec le catalyseur SA1.

## Revendications

1. Procédé d'oligomérisation des oléfines dans lequel le catalyseur d'oligomérisation comprend un support non-zéolithique à base de silice-alumine contenant une quantité supérieure à 5 % poids et inférieure ou égale à 95 % poids de silice (SiO₂) et possède les caractéristiques suivantes :
- un diamètre moyen poreux, mesuré par porosimétrie au mercure, compris entre 20 et 140 Å,
- un volume poreux total, mesuré par porosimétrie au mercure, compris entre 0,1 ml/g et 0,6 ml/g,
- un volume poreux total, mesuré par porosimétrie azote, compris entre 0,1 ml/g et 0,6 ml/g,
- une surface spécifique BET comprise entre 100 et 550 m²/g,
- un volume poreux, mesuré par porosimétrie au mercure, compris dans les pores de diamètre supérieurs à 140 Å inférieur à 0,1 ml/g,
- un volume poreux, mesuré par porosimétrie au mercure, compris dans les pores de diamètre supérieurs à 160 Å inférieur à 0,1 ml/g,
- un volume poreux, mesuré par porosimétrie au mercure, compris dans les pores de diamètre supérieurs à 200 Å inférieur à 0,1 ml/g,
- un volume poreux, mesuré par porosimétrie au mercure, compris dans les pores de diamètre supérieurs à 500 Å inférieur à 0, 1 ml/g, de préférence inférieur à 0.05 ml/g, de manière très préférée inférieur à 0.02 ml/g et de manière encore plus préférée inférieur à 0.01 ml/g et
- un diagramme de diffraction X qui contient au moins les raies principales caractéristiques d'au moins une des alumines de transition comprise dans le groupe composé par les alumines alpha, rhô, khi, êta, gamma, kappa, thêta et delta
- et le catalyseur silico-aluminique est obtenu à partir d'un mélange à quelque étape que ce soit d'un composé d'alumine partiellement soluble en milieu acide avec un composé de silice totalement soluble ou avec une combinaison totalement soluble d'alumine et de silice hydratées, mise en forme suivie d'un traitement hydrothermal ou thermique.

2. Procédé selon la revendication 1 dans lequel le catalyseur possède une distribution poreuse telle que
- le rapport entre le volume V2, mesuré par porosimétrie au mercure, compris entre le D_{moyen} -30 Å et le D_{moyen} +30 Å sur le volume mercure total est supérieur à 0,6,
- le volume V3, mesuré par porosimétrie au mercure, compris dans les pores de diamètre supérieur à D_{moyen} +30 Å est inférieur à 0,1 ml/g et
- le volume V6, mesuré par porosimétrie au mercure, compris dans les pores de diamètre supérieur à D_{moyen} +15 Å est inférieur à 0,2 ml/g.

3. Procédé selon l'une des revendications 1 à 2 tel que le support de catalyseur comprend au moins deux zones silico-aluminiques ayant des rapports Si/Al inférieurs ou supérieurs au rapport Si/Al global déterminé par fluorescence X.

4. Procédé selon l'une des revendications 1 à 3 tel que le support de catalyseur comprend une seule zone silico-aluminique ayant un rapport Si/Al égal au rapport Si/Al global déterminé par fluorescence X et inférieur à 2,3.

5. Procédé selon l'une des revendications précédentes dans lequel le catalyseur comprend au moins un élément métallique choisi parmi les éléments des groupes IVB, VB et VIB, ainsi que dans la première série du groupe VIII de la Classification Périodique des Éléments.

6. Procédé selon l'une des revendications précédentes dans lequel les oléfines proviennent d'une ou plusieurs sources choisies parmi les unités de craquage catalytique, de vapocraquage, de déshydrogénation de paraffines, de déshydratation polymérisante de méthanol en eau et oléfines légères, et toute autre source d'oléfines légères, et la charge est éventuellement diluée par un ajout de paraffines provenant d'une autre source, de même poids moléculaire et/ou plus lourdes que la charge oléfinique, aliphatiques ou cycliques, et la coupe oléfinique envoyée sur le catalyseur d'oligomérisation est débarrassée d'impuretés, telles que l'eau, les dérivés soufrés, les dérivés azotés basiques, avant d'être introduite dans le réacteur d'oligomérisation

7. Procédé selon l'une des revendications précédentes comprenant les étapes suivantes :on soumet une coupe C4 oléfinique à une oligomérisation en limitant la conversion globale des n-butènes à moins de 10 %, alors que plus de 90 % de la quantité d'isobutène est convertie en dimères et trimères , ladite réaction d'oligomérisation est effectuée à une température entre 30 et 300°C, sous une pression comprise entre 0,1 et 20 MPa et un volume de charge hydrocarbonée envoyé par volume de catalyseur et par heure comprise entre 0,05 et 5 h⁻¹ et
• on soumet l'effluent d'oligomérisation à une distillation pour obtenir une fraction (effluent léger) qui contient à plus de 90 % poids du butane, de l'isobutane et les butènes qui n'ont pas réagi lors de l'oligomérisation, une partie au moins de cette fraction alimentant ensuite une unité d'alkylation ou une unité d'hydratation, et une autre fraction constituée des oligomères obtenus utilisée ensuite comme base essence.

8. Procédé selon la revendication 7 dans lequel les oligomères obtenus sont réinjectés dans un réacteur d'oligomérisation supplémentaire.

9. Procédé selon l'une des revendications 7 à 8 dans lequel l'effluent léger d'oligomérisation est introduit dans un réacteur d'hydroisomérisation.

10. Procédé selon l'une des revendications 1 à 6 où on soumet une coupe C4 oléfinique ou C3-C4 oléfinique à une oligomérisation de telle sorte que moins de 80 % des butènes contenus dans la charge est converti et au moins 80 % de l'isobutène contenu dans la charge est converti en dimères ou trimères, la réaction d'oligomérisation se déroule à une température entre 40 et 250°C, une pression entre 0,1 et 10 MPa, et un volume de charge hydrocarbonée envoyé par volume de catalyseur et par heure entre 0,05 et 5 h⁻¹.

11. Procédé selon l'une des revendications 10 dans lequel l'isobutène a été au préalable éliminé en grande partie de la charge oléfinique.

12. Procédé selon l'une des revendications 1 à 6 comprenant les étapes suivantes :
• une première étape d'oligomérisation, dans laquelle on traite une coupe C4 ou C3-C4 oléfinique, dans un premier réacteur d'oligomérisation dans lequel la conversion globale des n-butènes contenus dans la charge est inférieure à 45 % et la conversion de l'isobutène est supérieure à 80 %, les oligomères obtenus étant des dimères et trimères à plus de 80 %,
• on envoie l'effluent de la première étape d'oligomérisation dans une colonne de fractionnement de façon à récupérer une première fraction contenant l'isobutène et les n-butènes non-convertis et une deuxième fraction consistant à 90 % en dimères et trimères de la réaction d'oligomérisation.
• deuxième étape d'oligomérisation, dans laquelle la première fraction récupérée est introduite dans un deuxième réacteur d'oligomérisation dans lequel au moins 50 % des n-butènes sont convertis et
• on envoie l'effluent de la deuxième étape d'oligomérisation dans la colonne de fractionnement associée au premier réacteur d'oligomérisation ou dans une deuxième colonne
et les réactions d'oligomérisation se déroulent à une température comprise entre 40 et 250°C, une pression comprise entre 0,1 et 10 MPa, et une quantité de charge hydrocarbonée envoyée par volume de catalyseur et par heure comprise entre 0,05 et 5 h⁻¹.

13. Procédé selon l'une des revendications 1 à 6 dans lequel on soumet une coupe C4 oléfinique ou une coupe C3C4 oléfinique, à une oligomérisation de telle sorte qu'au moins 90 % des 1-butène, au moins 80 % des 2-butènes et au moins 97 % de l'isobutène soient convertis, la réaction d'oligomérisation se déroule à une température comprise entre 60 et 250°C, une pression comprise entre 0,1 et 10 MPa, et une quantité de charge hydrocarbonée envoyée par volume de catalyseur et par heure comprise entre 0,05 et 5 h⁻¹.

14. Procédé selon l'une des revendications 1 à 6 dans lequel on soumet une coupe C3 oléfinique à une oligomérisation de telle sorte qu'au moins 80 % du propylène contenu dans la charge soit converti et la réaction d'oligomérisation est effectuée à une température entre 30 et 300°C, sous une pression entre environ 0,1 et 20 MPa et le volume de charge hydrocarbonée envoyé par volume de catalyseur et par heure se situe entre 0,05 et 5 h-1.

15. Procédé selon l'une des revendications précédentes dans lequel le diamètre moyen poreux du catalyseur mesuré par porosimétrie au mercure est compris entre 40 et 120 Å.

## Patentansprüche

1. Verfahren zur Oligomerisierung von Olefinen, wobei der Oligomerisierungskatalysator einen nicht-zeolithischen Träger auf der Basis von Siliciumdioxid-Aluminiumoxid umfasst, der eine Menge größer 5 Gew.-% und kleiner oder gleich 95 Gew.-%. Siliciumdioxid (SiO₂) umfasst und die folgenden Merkmale aufweist:
- einen mittleren Porendurchmesser, gemessen mittels Quecksilber-Porosimetrie, im Bereich zwischen 20 und 140 Å,
- ein Gesamtporenvolumen, gemessen mittels Quecksilber-Porosimetrie, im Bereich zwischen 0,1 ml/g und 0,6 ml/g,
- ein Gesamtporenvolumen, gemessen mittels Stickstoff-Porosimetrie, im Bereich zwischen 0,1 ml/g und 0,6 ml/g,
- eine spezifische BET-Oberfläche im Bereich zwischen 100 und 550 m²/g,
- ein Porenvolumen, gemessen mittels Quecksilber-Porosimetrie, im Bereich der Poren mit einem Durchmesser größer als 140 Å von kleiner als 0,1 ml/g,
- ein Porenvolumen, gemessen mittels Quecksilber-Porosimetrie, im Bereich der Poren mit einem Durchmesser größer als 160 Å von kleiner als 0,1 ml/g,
- ein Porenvolumen, gemessen mittels Quecksilber-Porosimetrie, im Bereich der Poren mit einem Durchmesser größer als 200 Å von kleiner als 0,1 ml/g,
- ein Porenvolumen, gemessen mittels Quecksilber-Porosimetrie, im Bereich der Poren mit einem Durchmesser größer als 500 Å von kleiner als 0,1 ml/g, bevorzugt kleiner als 0,05 ml/g, ganz besonders bevorzugt kleiner als 0,02 ml/g und noch stärker bevorzugt 0,01 ml/g und
- ein Röntgenbeugungsdiagramm, das mindestens die charakteristischen Hauptlinien von mindestens einem der Übergangsaluminiumoxide enthält, die die Gruppe, bestehend aus den alpha-, rho-, chi-, eta-, gamma-, kappa-, theta- und delta-Aluminiumoxiden, umfasst,
- und der Siliciumdioxid-Aluminiumoxid-Katalysator, egal in welchem Schritt, aus einer Mischung aus einer teilweise in saurem Medium löslichen Aluminiumoxidverbindung und einer vollständig löslichen Siliciumdioxidverbindung oder aus einer vollständig löslichen Kombination aus hydratisiertem Aluminiumoxid und hydrotiertem Siliciumdioxid erhalten wird, wobei auf die Formgebung eine hydrothermische oder thermische Behandlung folgt.

2. Verfahren nach Anspruch 1, wobei der Katalysator eine derartige Porenverteilung aufweist, dass
- das Verhältnis zwischen dem Volumen V2, gemessen mittels Quecksilber-Porosimetrie, im Bereich zwischen Dₘᵢₜₜₑₗ -30 Å und Dₘᵢₜₜₑₗ +30 Å zum gesamten Quecksilbervolumen größer als 0,6 ist,
- das Volumen V3, gemessen mittels Quecksilber-Porosimetrie, im Bereich der Poren mit einem Durchmesser größer als Dₘᵢₜₜₑₗ +30 kleiner als 0,1 ml/g ist, und
- das Volumen V6, gemessen mittels Quecksilber-Porosimetrie, im Bereich der Poren mit einem Durchmesser größer als Dₘᵢₜₜₑₗ +15 Å kleiner als 0,2 ml/g ist.

3. Verfahren nach einem der Ansprüche 1 bis 2, wobei der Katalysatorträger mindestens zwei Siliciumdioxid-Aluminiumoxid-Zonen umfasst, die Si/Al-Verhältnisse aufweisen, die kleiner oder größer sind als das Si/Al-Gesamtverhältnis, das durch Röntgenfluoreszenz bestimmt wird.

4. Verfahren nach einem der Ansprüche 1 bis 3, wobei der Katalysatorträger eine einzige Siliciumdioxid-Aluminiumoxid-Zone umfasst, die ein Si/Al-Verhältnis gleich dem Si/Al-Gesamtverhältnis aufweist, das durch Röntgenfluoreszenz bestimmt wird, und kleiner als 2,3 ist.

5. Verfahren nach einem der vorhergehenden Ansprüche, wobei der Katalysator mindestens ein Metallelement umfasst, ausgewählt aus den Elementen der Gruppen IVB, VB und VIB, sowie aus der ersten Serie der Gruppe VIII des Periodensystems der Elemente.

6. Verfahren nach einem der vorhergehenden Ansprüche, wobei die Olefine aus einer oder mehreren Quellen stammen, ausgewählt aus Einheiten zum katalytischen Cracken, zum Dampfcracken, zur Dehydrierung von Paraffinen, zur polymerisierenden Dehydrierung von Methanol in Wasser und leichte Olefine, und jeder anderen Quelle für leichte Olefine, und die Beschickung gegebenenfalls durch eine Zugabe von Paraffinen verdünnt wird, die aus einer anderen Quelle stammen, mit gleichem Molekulargewicht und/oder schwerer als die olefinischen, aliphatischen oder cyclischen Beschickungen, und der Olefinschnitt, der auf den Oligomerisierungskatalysator geschickt wird, frei von Verunreinigungen ist, wie beispielsweise Wasser, schwefelhaltigen Derivaten, basischen, stickstoffhaltigen Derivaten, bevor sie in den Oligomerisierungsreaktor eingeführt wird.

7. Verfahren nach einem der vorhergehenden Ansprüche, das die folgenden Schritte umfasst: Oligomerisieren eines C4-Olefinschnitts, wobei die Gesamtumwandlung der n-Butene auf weniger als 10 % begrenzt wird, während mehr als 90 % der Isobutenmenge in Dimere und Trimere umgewandelt werden, wobei die Oligomerisierungsreaktion bei einer Temperatur zwischen 30 und 300 °C, unter einem Druck im Bereich zwischen 0,1 und 20 MPa und mit einem Kohlenwasserstoffbeschickungsvolumen, das pro Katalysatorvolumen und pro Stunde zugeführt wird, im Bereich zwischen 0,05 und 5 h⁻¹ durchgeführt wird,
- Destillieren des Oligomerisierungsabflusses, um eine Fraktion (leichter Abfluss) zu erhalten, die mehr als 90 Gew.-% Butan, Isobutan und die Butene enthält, die während der Oligomerisierung nicht umgesetzt wurden, wobei mindestens ein Teil dieser Fraktion anschließend eine Alkylierungs- oder eine Hydrierungseinheit speist, und eine andere Fraktion, die aus den erhaltenen Oligomeren besteht, anschließend als Benzingrundstoff verwendet wird.

8. Verfahren nach Anspruch 7, wobei die erhaltenen Oligomere in einen zusätzlichen Oligomerisierungsreaktor zurückgeführt werden.

9. Verfahren nach einem der Ansprüche 7 bis 8, wobei der leichte Oligomerisierungsabfluss in einen Hydroisomerisierungsreaktor eingeführt wird.

10. Verfahren nach einem der Ansprüche 1 bis 6, wobei ein C4-Olefin- oder C3-C4-Olefinschnitt derart oligomerisiert wird, dass weniger als 80 % der Butene, die in der Beschickung enthalten sind, umgewandelt werden und mindestens 80 % des Isobutens, das in der Beschickung enthalten ist, in Dimere oder Trimere umgewandelt werden, wobei die Oligomerisierungsreaktion bei einer Temperatur zwischen 40 und 250 °C, einem Druck zwischen 0,1 und 10 MPa und einem Kohlenwasserstoffbeschickungsvolumen, das pro Katalysatorvolumen und pro Stunde zugeführt wird, zwischen 0,05 und 5 h⁻¹, abläuft

11. Verfahren nach einem der Ansprüche 10, wobei das Isobuten größtenteils aus der Olefinbeschickung entfernt wurde.

12. Verfahren nach einem der Ansprüche 1 bis 6, das die folgenden Schritte umfasst:
• einen ersten Oligomerisierungsschritt, wobei ein C4- oder C3-C4-Olefinschnitt in einem ersten Oligomerisierungsreaktor behandelt wird, wobei die Gesamtumwandlung der n-Butene, die in der Beschickung enthalten sind, weniger als 45 % beträgt und die Umwandlung des Isobutens mehr als 80 % beträgt, wobei die erhaltenen Oligomere zu mehr als 80 % Dimere und Trimere sind,
• der Abfluss aus dem ersten Oligomerisierungsschritt in eine Fraktionierkolonne geschickt wird, um eine erste Fraktion, die das Isobuten und die nicht umgewandelten n-Butene enthält, und ein zweite Fraktion, die zu 90 % aus Dimeren und Trimeren der Oligomerisierungsreaktion besteht, zurückzugewinnen,
• einen zweiten Oligomerisierungsschritt, wobei die erste zurückgewonnene Fraktion in einen zweiten Oligomerisierungsreaktor eingeführt wird, in dem mindestens 50 % der n-Butene umgewandelt werden, und
• der Abfluss aus dem zweiten Oligomerisierungsschritt in die Fraktionierkolonne, die mit dem ersten Oligomerisierungsreaktor verbunden ist, oder in eine zweite Kolonne geschickt wird,
und die Oligomerisierungsreaktionen bei einer Temperatur im Bereich zwischen 40 und 250 °C, einem Druck im Bereich zwischen 0,1 und 10 MPa und einer Kohlenwasserstoffbeschickungsmenge, die pro Katalysatorvolumen und pro Stunde zugeführt wird, zwischen 0,05 und 5 h⁻¹ ablaufen.

13. Verfahren nach einem der Ansprüche 1 bis 6, wobei ein C4-Olefinschnitt oder ein C3-C4-Olefinschnitt derart oligomerisiert werden, dass mindestens 90 % der 1-Butene, mindestens 80 % der 2-Butene und mindestens 97 % des Isobutens umgewandelt werden, wobei die Oligomerisierungsreaktion bei einer Temperatur im Bereich zwischen 60 und 250 °C, einem Druck im Bereich zwischen 0,1 und 10 MPa und einer Kohlenwasserstoffbeschickungsmenge, die pro Katalysatorvolumen und pro Stunde zugeführt wird, im Bereich zwischen 0,05 und 5 h⁻¹ abläuft.

14. Verfahren nach einem der Ansprüche 1 bis 6, wobei ein C3-Olefinschnitt derart oligomerisiert wird, dass mindestens 80 % des in der Beschickung enthaltenen Propylens umgewandelt werden und die Oligomerisierungsreaktion bei einer Temperatur zwischen 30 und 300 °C, unter einem Druck zwischen etwa 0,1 und 20 MPa durchgeführt wird und das Kohlenwasserstoffbeschickungsvolumen, das pro Katalysatorvolumen und pro Stunde zugeführt wird, zwischen 0,05 und 5 h⁻¹ liegt.

15. Verfahren nach einem der vorhergehenden Ansprüche, wobei der mittlere Porendurchmesser des Katalysators, gemessen mittels Quecksilber-Porosimetrie, im Bereich zwischen 40 und 120 Å liegt.

## Claims

1. A process for oligomerizing olefins in which the oligomerization catalyst comprises a non zeolitic support based on silica-alumina containing a quantity of more than 5% by weight and 95% by weight or less of silica (SiO₂) and has the following characteristics:
• a mean pore diameter, measured by mercury porosimetry, in the range 20 to 140 Å;
• a total pore volume, measured by mercury porosimetry, in the range 0.1 ml/g to 0.6 ml/g;
• a total pore volume, measured by nitrogen porosimetry, in the range 0.1 ml/g to 0.6 ml/g;
• a BET specific surface area in the range 100 to 550 m²/g;
• a pore volume, measured by mercury porosimetry, included in pores with a diameter of more than 140 Å, of less than 0.1 ml/g;
• a pore volume, measured by mercury porosimetry, included in pores with a diameter of more than 160 Å, of less than 0.1 ml/g;
• a pore volume, measured by mercury porosimetry, included in pores with a diameter of more than 200 Å, of less than 0.1 ml/g;
• a pore volume, measured by mercury porosimetry, included in pores with a diameter of more than 500 Å, of less than 0.1 ml/g, preferably less than 0.05 ml/g, more preferably less than 0.02 ml/g and still more preferably less than 0.01 ml/g; and
• an X ray diffraction diagram containing at least the principal characteristic peaks of at least one of the transition aluminas included in the group composed of alpha, rho, khi, eta, gamma, kappa, theta and delta aluminas,
• and the silica-alumina catalysts obtained by mixing, at any stage, an alumina compound which is partially soluble in an acidic medium with a silica compound which is completely soluble or with a completely soluble combination of alumina and hydrated silica, then shaping, followed by hydrothermal or thermal treatment.

2. A process according to claim 1, in which the catalyst has a pore distribution such that:
• the ratio between the volume V2, measured by mercury porosimetry, in the range between Dₘₑₐₙ - 30 Å and Dₘₑₐₙ + 30 Å, to the total mercury volume is more than 0.6;
• the volume V3, measured by mercury porosimetry, in pores with diameters of more than Dₘₑₐₙ + 30 Å, is less than 0.1 ml/g; and
• the volume V6, measured by mercury porosimetry, in pores with diameters of more than Dₘₑₐₙ + 15 Å, is less than 0.2 ml/g.

3. A process according to one of claims 1 to 2, in which the catalyst support comprises at least two silica-alumina zones having Si/Al ratios which are higher or lower than the overall Si/Al ratio determined by X ray fluorescence.

4. A process according to one of claims 1 to 3, in which the catalyst support comprises a single silica-alumina zone having a Si/Al ratio equal to the overall Si/Al ratio determined by X ray fluorescence and less than 2.3.

5. A process according to one of the preceding claims, in which the catalyst comprises at least one metallic element selected from elements from groups IVB, VB and VIB as well as from the first series of group VIII of the periodic table.

6. A process according to one of the preceding claims, in which the olefins derive from one or more sources selected from catalytic cracking units, steam cracking units, paraffin dehydrogenation units, units for polymerizing dehydration of methanol to water and light olefins, and any other source of light olefins and which the feed is diluted by adding aliphatic or cyclic paraffins deriving from another source, having the same molecular weight and/or being heavier than the olefinic feed and the olefinic cut sent to the oligomerization catalyst is free of impurities such as water, sulphur-containing derivatives or basic nitrogen-containing derivatives before being introduced into the oligomerization reactor.

7. A process according to one of the preceding claims, comprising the following steps:
• oligomerizing an olefinic C4 cut, limiting the overall conversion of n-butenes to less than 10% while more than 90% of the quantity of isobutene is converted into dimers and trimmers, the oligomerization reaction is carried out at a temperature between 30°C and 300°C, at a pressure in the range 0.1 to 20 MPa and with a volume of hydrocarbon feed per volume of catalyst per hour in the range 0.05 to 5 h⁻¹, and
• distilling the oligomerization effluent to obtain a fraction (light effluent) which contains more than 90% by weight of butane, isobutane and butenes which have not reacted during the oligomerization, at least a portion of said fraction then supplying an alkylation unit or a hydration unit, and another fraction constituted by the oligomers obtained subsequently used as a gasoline base.

8. A process according to claim 7, in which the oligomers obtained are re-injected into a supplementary oligomerization reactor.

9. A process according to one of claims 7 to 8, in which the light oligomerization effluent is introduced into a hydrosomerization reactor.

10. A process according to one of claims 1 to 6, in which an olefinic C4 cut or an olefinic C3-C4 cut undergoes oligomerization such that less than 80% of the butenes contained in the feed is converted and at least 80% of the isobutene contained in the feed is converted into dimers or trimmers, the oligomerization reaction is carried out at a temperature between 40°C and 250°C, a pressure between 0.1 and 10 MPa and with a volume of hydrocarbon feed per volume of catalyst per hour between 0.05 and 5 h⁻¹.

11. A process according to claim 10, in which the isobutene has been substantially eliminated from the olefinic feed in advance.

12. A process according to one of claims 1 to 6, comprising the following steps:
• a first oligomerization step, in which an olefinic C4 or C3-C4 cut is treated in a first oligomerization reactor in which the overall conversion of n-butenes contained in the feed is less than 45% and the isobutene conversion is more than 80%, the oligomers obtained being more than 80% dimers and trimers;
• the effluent from the first oligomerization step is sent to a fractionation column to recover a first fraction containing isobutene and unconverted n-butenes and a second fraction consisting of 90% dimers and trimers from the oligomerization reaction;
• a second oligomerization step, in which the first recovered fraction is introduced into a second oligomerization reactor in which at least 50% of the n-butenes are converted; and
• the effluent from the second oligomerization step is sent to the fractionation column associated with the first oligomerization reactor or to a second column,
the oligomerization reactions are carried out at a temperature in the range 40°C to 250°C, at a pressure in the range 0.1 to 10 MPa, and with a quantity of hydrocarbon feed per volume of catalyst per hour in the range 0.05 to 5 h-1.

13. A process according to one of claims 1 to 6, in which an olefinic C4 cut or an olefinic C3-C4 cut undergoes oligomerization such that at least 90% of the 1-butene, at least 80% of the 2-butenes and at least 97% of the isobutene are converted, the oligomerization reaction is carried out at a temperature in the range 60°C to 250°C, at a pressure in the range 0.1 to 10 MPa, and with a quantity of hydrocarbon feed per volume of catalyst per hour in the range 0.05 to 5 h⁻¹.

14. A process according to one of claims 1 to 6, in which an olefinic C3 cut undergoes oligomerization such that at least 80% of the propylene contained in the feed is converted, the oligomerization reaction is carried out at a temperature between 30°C and 300°C, at a pressure in the range about 0.1 to 20 MPa, and with a volume of hydrocarbon feed per volume of catalyst per hour in the range 0.05 to 5 h⁻¹.

15. A process according to one of the preceding claims, in which the mean pore diameter of the catalyst, measured by mercury porosimetry, is in the range 40 to 120 Å.
